# EUROPEAN PATENT SPECIFICATION

(11) **EP 1 678 296 B1**
(45) Date of publication and mention of the grant of the patent: **13.07.2011**
(21) Application number: 04790060.0
(22) Date of filing: 22.10.2004
(51) Int. Cl.: C12N 9/58, C12N 9/76, C11D 3/386

(54) **PROTEASE WITH IMPROVED STABILITY IN DETERGENTS**
PROTEASE MIT VERBESSERTER STABILITÄT IN DETERGENTIEN
PROTEASE A STABILITE AMELIOREE DANS LES DETERGENTS

(30) Priority: 23.10.2003 DK 200301562
(43) Date of publication of application: 12.07.2006
(73) Proprietor: Novozymes A/S, 2880 Bagsvaerd (DK)
(72) Inventor: WU, Wenping, Er Dan Yuan, Bldg 3, Room 13, Haidian District, Beijing 100085 (CN); JOKUMSEN, Kirsten, Vaever, DK-2970 Hoersholm (DK); STRINGER, Mary, Ann, DK-2860 Soeborg (DK)
(86) International application number: PCT/DK2004/000730
(87) International publication number: WO 2005/040372

(56) References cited:
- WO-A-88/07581
- WO-A-89/04361
- WO-A-94/25583
- WO-A-95/30743
- OLIVIERI FLORENCIA ET AL: "Characterization of an extracellular serine protease of Fusarium eumartii and its action on pathogenesis related proteins" EUROPEAN JOURNAL OF PLANT PATHOLOGY, vol. 108, no. 1, January 2002 (2002-01), pages 63-72, XP008041158 ISSN: 0929-1873

### SEQUENCE LISTING

<110> Novozymes A/S
<120> Trypsin like protease
<130> 10178
<160> 2
<170> PatentIn version 3.2
<210> 1
   <211> 1004
   <212> DNA
   <213> Fusarium solani
<220>
   <221> CDS
   <222> (52) .. (804)
<220>
   <221> mat_peptide
   <222> (127) .. (804)
   <223> 52-102: signal peptide and 53-126: pro-peptide
<400> 1
   ctcttcactc ttcaactctc tactcttgga atcccctgtc tgctcttcac c atg gtc      57
                                                            Met Val
                                                            -25
   aag ttt gct gcc atc ctc gca ctt gtt gcg cct ctt gtc gcc gct cgg      105
   Lys Phe Ala Ala Ile Leu Ala Leu Val Ala Pro Leu Val Ala Ala Arg
               -20                 -15                 -10
   cct cag gac tca tca ccc atg atc gtt ggt gga act gct gcc agc gct      153
   Pro Gln Asp Ser Ser Pro Met Ile Val Gly Gly Thr Ala Ala Ser Ala
           -5              -1  1               5
   ggt gac ttc ccc ttc atc gtc agc atc gcc tac aat ggt ggc cct tgg      201
   Gly Asp Phe Pro Phe Ile Val Ser Ile Ala Tyr Asn Gly Gly Pro Trp
   10                  15                  20                  25
   tgc gga ggt acc ctc ctc aac gcc aac acc gtc atg act gct gcc cac      249
   Cys Gly Gly Thr Leu Leu Asn Ala Asn Thr Val Met Thr Ala Ala His
                   30                  35                  40
   tgc acc caa ggt cgc tct gct agc gcc ttc cag gtc cgc gcc gga agt      297
   Cys Thr Gln Gly Arg Ser Ala Ser Ala Phe Gln Val Arg Ala Gly Ser
               45                  50                  55
   ctg aac cgc aac tcg ggt ggt gtt acc tct tcc gtt tct tcc atc agg      345
   Leu Asn Arg Asn Ser Gly Gly Val Thr Ser Ser Val Ser Ser Ile Arg
           60                  65                  70
   atc cat cct agc ttc agt agc tcg acc ctg aac aac gat gtt tcc atc      393
   Ile His Pro Ser Phe Ser Ser Ser Thr Leu Asn Asn Asp Val Ser Ile
       75                  80                  85
   ctg aag ctg tcc acc ccc atc tcg act agc tcc act att tct tat ggt      441
   Leu Lys Leu Ser Thr Pro Ile Ser Thr Ser Ser Thr Ile Ser Tyr Gly
   90                  95                  100                 105
   cgc ctg gct gcg tcg ggc tct gac cct gtt gcc ggc tct gat gcc aca      489
   Arg Leu Ala Ala Ser Gly Ser Asp Pro Val Ala Gly Ser Asp Ala Thr
                   110                 115                 120
   gtt gct ggc tgg ggt gtc act tct cag ggc tct tcc agc tct ccc gtg      537
   Val Ala Gly Trp Gly Val Thr Ser Gln Gly Ser Ser Ser Ser Pro Val
               125                 130                 135
   gct ttg agg aag gtt acc att ccc atc gtc tcc cgc acc act tgc cga      585
   Ala Leu Arg Lys Val Thr Ile Pro Ile Val Ser Arg Thr Thr Cys Arg
           140                 145                 150
   tcc cag tat ggc act tct gcc atc acc acc aac atg ttc tgc gct ggt      633
   Ser Gln Tyr Gly Thr Ser Ala Ile Thr Thr Asn Met Phe Cys Ala Gly
       155                 160                 165
   ctt gct gag ggt ggt aag gac tct tgc cag ggc gac agc ggc ggt ccc      681
   Leu Ala Glu Gly Gly Lys Asp Ser Cys Gln Gly Asp Ser Gly Gly Pro
   170                 175                 180                 185
   att gtc gat acc tcc aac act gtc att ggc att gtt tct tgg ggt gag      729
   Ile Val Asp Thr Ser Asn Thr Val Ile Gly Ile Val Ser Trp Gly Glu
                   190                 195                 200
   ggt tgt gct cag ccc aac tta tct ggt gtc tat gcc cga gtt gga tct      777
   Gly Cys Ala Gln Pro Asn Leu Ser Gly Val Tyr Ala Arg Val Gly Ser
               205                 210                 215
   ctc cgc act tac atc gac ggc cag ctg taaattgctc ggtcggttgg            824
   Leu Arg Thr Tyr Ile Asp Gly Gln Leu
           220                 225
   tttacatttc tgttctaggc agtttgcttg tcagagactt ttgttgagat ggggacggaa    884
   gatggagtag gaatgctgag agtgtttgtt tgagagttta gttgatagtc aagatccaag    944
   tcccggttgg ccatgcactg gcaactcgag cgactagatt ctcaataaaa ttgttcgtgg   1004
<210> 2
   <211> 251
   <212> PRT
   <213> Fusarium solani
<400> 2
   Met Val Lys Phe Ala Ala Ile Leu Ala Leu Val Ala Pro Leu Val Ala
   -25                 -20                 -15                 -10
   Ala Arg Pro Gln Asp Ser Ser Pro Met Ile Val Gly Gly Thr Ala Ala
                   -5              -1  1               5
   Ser Ala Gly Asp Phe Pro Phe Ile Val Ser Ile Ala Tyr Asn Gly Gly
           10                  15                  20
   Pro Trp Cys Gly Gly Thr Leu Leu Asn Ala Asn Thr Val Met Thr Ala
       25                  30                  35
   Ala His Cys Thr Gln Gly Arg Ser Ala Ser Ala Phe Gln Val Arg Ala
   40                  45                  50                  55
   Gly Ser Leu Asn Arg Asn Ser Gly Gly Val Thr Ser Ser Val Ser Ser
                   60                  65                  70
   Ile Arg Ile His Pro Ser Phe Ser Ser Ser Thr Leu Asn Asn Asp Val
               75                  80                  85
   Ser Ile Leu Lys Leu Ser Thr Pro Ile Ser Thr Ser Ser Thr Ile Ser
           90                  95                  100
   Tyr Gly Arg Leu Ala Ala Ser Gly Ser Asp Pro Val Ala Gly Ser Asp
       105                 110                 115
   Ala Thr Val Ala Gly Trp Gly Val Thr Ser Gln Gly Ser Ser Ser Ser
   120                 125                 130                 135
   Pro Val Ala Leu Arg Lys Val Thr Ile Pro Ile Val Ser Arg Thr Thr
                   140                 145                 150
   Cys Arg Ser Gln Tyr Gly Thr Ser Ala Ile Thr Thr Asn Met Phe Cys
               155                 160                 165
   Ala Gly Leu Ala Glu Gly Gly Lys Asp Ser Cys Gln Gly Asp Ser Gly
           170                 175                 180
   Gly Pro Ile Val Asp Thr Ser Asn Thr Val Ile Gly Ile Val Ser Trp
       185                 190                 195
   Gly Glu Gly Cys Ala Gln Pro Asn Leu Ser Gly Val Tyr Ala Arg Val
   200                 205                 210                 215
   Gly Ser Leu Arg Thr Tyr Ile Asp Gly Gln Leu
                   220                 225

## Description

### TECHNICAL FIELD

The present invention relates to proteases having improved stability in detergent compositions. The present invention also relates to isolated polynucleotides encoding the proteases, nucleic acid constructs, recombinant expression vectors, host cells comprising the nucleic acid constructs, and methods for producing and using the proteases of the invention. Further, the present invention relates to cleaning and detergent compositions comprising the proteases of the invention as well as to use of such protease in detergent compositions.

### BACKGROUND OF THE INVENTION

In the detergent industry enzymes have for more than 30 years been implemented in washing formulations. Enzymes used in such formulations comprise proteases, lipases, amylases, cellulases, mannosidases as well as other enzymes or mixtures thereof. Commercially most important enzymes are proteases.

A high number of such protease variants are disclosed in the art, such as in EP 130756 (GENENTECH)(corresponding to US Reissue Patent No. 34,606 (GENENCOR)); EP 214435 (HENKEL); WO95/30743 (Novo Nordisk) WO 87/04461 (AMGEN); WO 87/05050 (GENEX); EP 260105 (GENENCOR); Thomas, Russell, and Fersht (1985) Nature 318 375-376; Thomas, Russell, and Fersht (1987) J. Mol. Biol. 193 803-813; Russel and Fersht Nature 328 496-500 (1987); WO 88/08028 (Genex); WO 88/08033 (Amgen); WO 95/27049 (SOLVAY S.A.); WO 95/30011 (PROCTER & GAMBLE COMPANY); WO 95/30010 (PROCTER & GAMBLE COMPANY); WO 95/29979 (PROCTER & GAMBLE COMPANY); US 5.543.302 (SOLVAY S.A.); EP 251 446 (GENENCOR); WO 89/06279 (NOVOZYMES A/S); WO 91/00345 (NOVOZYMES A/S); EP 525 610 A1 (SOLVAY); WO 94/02618 (GIST-BROCADES N.V.).

WO 89/06270 (Novozymes A/S) discloses a detergent composition comprising a protease with a narrow substrate specificity, namely a trypsin-like protease capable of cleaving peptide bonds at C-terminal side of lysine or arginine.

Further WO 94/25583 discloses the cloning of a DNA sequence encoding a Fusarium trypsin-like protease and obtaining expression of an active trypsin-like protease from said DNA-sequence.

However, even though a number of useful proteases and protease variants have been described, there is still a need for further improvement of proteases or protease variants for a number of industrial uses.

In particular, the problem of stability in detergents has been pronounced due to the fact that substances present in the detergent tend to reduce the stability of proteases.

Therefore, an object of the present invention is to provide improved proteases, which are suitable for use in detergents for the use in for example laundry and/or cleaning of hard surfaces.

### SUMMARY OF THE INVENTION

In a first aspect the present invention relates to a protease having improved stability in detergent, the protease being selected from the group consisting of
a. a protease having an amino acid sequence which has at least 80% identity with the amino acid sequence shown as amino acids 1 to 226 of SEQ ID NO: 2;
b. a protease which is encoded by a nucleic acid sequence which has at least 80% identity with the nucleic acid sequence shown as nucleic acid 52-804 of SEQ ID NO:1,
where the protease - when tested in "Example V Stability in detergent" - has a residual activity of at least 50% after storage at 35°C.

In a second aspect the present invention relates to an isolated polynucleotide comprising a nucleic acid sequence that encodes for the proteases according to the invention.

In a third aspect the present invention relates to a nucleic acid construct comprising the nucleic acid sequence according to the invention operably linked to one or more control sequences capable of directing the expression of the protease in a suitable host.

In a fourth aspect the present invention relates to a recombinant expression vector comprising the nucleic acid construct according to the invention, a promoter, and transcriptional and trans-lational stop signals.

In a fifth aspect the present invention relates to a recombinant host cell comprising the nucleic acid construct of the invention.

In a sixth aspect the present invention relates to a method for producing the protease according to the invention, the method comprising:
(a) cultivating a recombinant host cell according to the invention under conditions conducive to the production of the protease; and
(b) recovering the protease.

In an seventh aspect the present invention relates to a cleaning or detergent composition, preferably a laundry or dish wash composition, comprising the protease according to the invention.

Further aspects of the present invention relate to use of the proteases according to the invention in a cleaning or detergent composition; a method for cleaning or washing a hard surface or laundry comprising contacting the hard surface or the laundry with the composition of the invention.

### DEFINITIONS

Prior to discussing this invention in further detail, the following terms and conventions will first be defined.

### NOMENCLATURE OF AMINO ACIDS

| | | | | |
|---|---|---|---|---|
| A | = | Ala | = | Alanine |
| V | = | Val | = | Valine |
| L | = | Leu | = | Leucine |
| I | = | Ile | = | Isoleucine |
| P | = | Pro | = | Proline |
| F | = | Phe | = | Phenylalanine |
| W | = | Trp | = | Tryptophan |
| M | = | Met | = | Methionine |
| G | = | Gly | = | Glycine |
| S | = | Ser | = | Serine |
| T | = | Thr | = | Threonine |
| C | = | Cys | = | Cysteine |
| Y | = | Tyr | = | Tyrosine |
| N | = | Asn | = | Asparagine |
| Q | = | Gln | = | Glutamine |
| D | = | Asp | = | Aspartic Acid |
| E | = | Glu | = | Glutamic Acid |
| K | = | Lys | = | Lysine |
| R | = | Arg | = | Arginine |
| H | = | His | = | Histidine |
| X | = | Xaa | = | Any amino acid |

### NOMENCLATURE OF NUCLEIC ACIDS

| | | | |
|---|---|---|---|
| A | = | Adenine | |
| G | = | Guanine | |
| C | = | Cytosine | |
| T | = | Thymine | (only in DNA) |
| U | = | Uracil | (only in RNA) |

### Proteases

Enzymes cleaving the amide linkages in protein substrates are classified as proteases, or (interchangeably) peptidases (see Walsh, 1979, Enzymatic Reaction Mechanisms. W.H. Freeman and Company, San Francisco, Chapter 3).

### Serine proteases

A serine protease is an enzyme which catalyzes the hydrolysis of peptide bonds, and in which there is an essential serine residue at the active site (White, Handler and Smith, 1973 "Principles of Biochemistry," Fifth Edition, McGraw-Hill Book Company, NY, pp. 271-272). The bacterial serine proteases have molecular weights in the 20,000 to 45,000 Dalton range. They are inhibited by diisopropyl fluorophosphate. They hydrolyze simple terminal esters and are similar in activity to eukaryotic chymotrypsin, also a serine protease. A more narrow term, alkaline protease, covering a sub group, reflects the high pH optimum of some of the serine proteases, from pH 9.0 to 11.0 (for review, see Priest (1977) Bacteriological Rev. 41 711-753).

### Trypsin-like protease

In the present context the term "trypsin-like protease" is intended to indicate an enzyme having an activity similar to that of trypsin, i.e. an enzyme capable of cleaving peptide bonds at the C-terminal side of lysine or arginine. The trypsin-like protease activity may be determined in an assay based on cleavage of a trypsin substrate e.g. as described in the Materials and Methods section below, example IV.

### Parent protease

A parent protease may be a protease isolated from a natural source, wherein subsequent modifications have been made while retaining the characteristic of a protease. Furthermore, a parent protease may also be a protease which has been prepared by the DNA shuffling technique, such as described by J.E. Ness et al., Nature Biotechnology, 17, 893-896 (1999).
Alternatively, the term "parent protease" may be termed "wild type protease".

### Modification(s) of a protease

The term "modification(s)" used herein is defined to include chemical modification of a protease as well as genetic manipulation of the DNA encoding a protease. The modification(s) can be replacement(s) of the amino acid side chain(s), substitution(s), deletion(s) and/or insertions in or at the amino acid(s) of interest.

### Protease variant

In the context of this invention, the term protease variant or mutated protease means a protease that has been produced by an organism which is expressing a mutant gene derived from a parent micro organism which possessed an original or parent gene and which produced a corresponding parent enzyme, the parent gene having been mutated in order to produce the mutant gene from which said mutated protease is produced when expressed in a suitable host. Analogously, the mutant gene may also be derived from a parent gene produced by DNA shuffling technique.

### Homologous protease sequences

In the present context, the homology between two amino acid sequences is described by the parameter "identity".
In order to determine the degree of identity between two proteases, the GAP routine of the AlignX application of the Vector NTI Program Suite v8 using default settings can be applied. The output from the routine is besides the amino acid alignment the calculation of the "Percent Identity" between the two sequences.
Based on this description it is routine for a person skilled in the art to identify suitable homologous proteases and corresponding homologous active site loop regions, which can be modified according to the invention.

### Isolated polynucleotide

The term "isolated polynucleotide" as used herein refers to a polynucleotide, which has been isolated and purified and is thus in a form suitable for use within genetically engineered protein production systems. Such isolated molecules may be those that are separated from their natural environment and include cDNA and genomic clones as well as polynucleotides derived from DNA shuffling experiments or from site-directed autogenesis experiments. Isolated polynucleotides of the present invention are free of other genes with which they are ordinarily associated, but may include 5' and 3' untranslated regions such as promoters and terminators. The identification of associated regions will be evident to one of ordinary skill in the art (see for example Dynan and Tijan, Nature 316:774-78, 1985). The term "isolated nucleic acid sequence" may alternatively be termed "isolated DNA sequence", "cloned nucleic acid sequence" or "cloned DNA sequence".

### Isolated protein

When applied to a protein, the term "isolated" indicates that the protein has been removed from its native environment.
In a preferred form, the isolated protein is substantially free of other proteins, particularly other homologous proteins (i.e. "homologous impurities" (see below)).
An isolated protein is more than 10% pure, preferably more than 20% pure, more preferably more than 30% pure, as determined by SDS-PAGE. Further, it is preferred to provide the protein in a highly purified form, i.e. more than 40% pure, more than 60% pure, more than 80% pure, more preferably more than 95% pure, and most preferably more than 99% pure, as determined by SDS-PAGE.

The term "isolated protein" may alternatively be termed "purified protein".

### Homologous impurities

The term "homologous impurities" means any impurity (e.g. another polypeptide than the protease of the invention), which originate from the homologous cell where the protease of the invention is originally obtained from.

### Obtained from

The term "obtained from" as used herein in connection with a specific microbial source means that the polynucleotide and/or protease produced by the specific source, or by a cell in which a gene from the source has been inserted.

### Substrate

The term "substrate" used in connection with a substrate for a protease should be interpreted in its broadest form as comprising a compound containing at least one peptide bond susceptible to hydrolysis by a protease.

### Product

The term "product" used in connection with a product derived from a protease enzymatic reaction should in the context of the present invention be interpreted to include the products of a hydrolysis reaction involving a protease. A product may be the substrate in a subsequent hydrolysis reaction.

### Wash Performance

In the present context, the term "wash performance" is used as an enzyme's ability to remove soil, in particular egg stains present on the object to the cleaned during e.g. wash or hard surface cleaning. See also the "Model Detergent Wash Performance Test" in Example VII.

### BRIEF DESCRIPTION OF THE DRAWING

Figure 1 describes the inhibition profile of the protease of the invention.
Figure 2 describes the substrate specificity of the protease of the invention.
Figure 3 describes the temperature profile of the protease of the invention.
Figure 4 describes the pH profile of the protease of the invention as determined by DMC-assay.
Figure 5 describes the pH profile of the protease of the invention as determined by ACZL-casein-assay.
Figure 6 describes the pH-stability profile of the protease of the invention.

### DETAILED DESCRIPTION OF THE INVENTION

In a first interesting aspect of the present invention, the trypsin like protease having improved stability is an isolated protease which has at least 80% identity with the amino acid sequence shown as amino acids 1 to 226 of SEQ ID NO: 2 (i.e. the mature protease).

In an interesting embodiment the protease of the invention has more than 85%, or more than 90%, or more than 92%, or more than 94%, or more than 96%, or more than 97%, or more than 98%, or more than 99% identity with the amino acid sequence shown as amino acids 1 to 226 of SEQ ID NO:2.

In a further interesting aspect the protease is a variant of a protease having the amino acid sequence shown as amino acids -25 to 226 of SEQ ID NO:2 comprising a substitution, deletion, and/or insertion of one or more amino acid residues.

Alignments of sequences and calculation of identity scores can be done using a full Smith-Waterman alignment, useful for both protein and DNA alignments. Amino acid sequences may be aligned with the AlignX application of the Vector NTI Program Suite v8 using default settings, which employ a modified ClustalW algorithm (Thompson, J.D., Higgins, D.G., and Gibson T.J., 1994), the blosum62mt2 score matrix, a gap opening penalty of 10 and a gap extension penalty of 0.1.

By performing such alignments between the amino acid sequences of the protease having the amino acid sequence of SEQ ID NO: 2 and the protease considered closest prior art, an identity of 73% of the mature protein was found.

In another interesting embodiment of the invention the isolated protease is encoded by a nucleic acid sequence which hybridises under low stringency conditions, preferably under medium stringency conditions, more preferably under high stringency conditions with (i) a complementary strand of the nucleic acid sequence shown as nucleotides 127 to 804 of SEQ ID NO: 1, or (ii) a subsequence of (i) of at least 100 nucleotides (J. Sambrook, E.F. Fritsch, and T. Maniatus, 1989, Molecular Cloning, A Laboratory Manual, 2d edition, Cold Spring Harbor, New York).

The subsequence of the complementary strand of the nucleic acid sequence shown as nucleotides 127 to 804 of SEQ ID NO: 1 may be at least 100 nucleotides or preferably at least 200 nucleotides, or at least 300 nucleotides, or at least 400 nucleotides. Moreover, the subsequence should encode a protease fragment, which has proteolytic activity. The protease may also be allelic variants or fragments of the protease that have proteolytic activity.

The nucleic acid sequence of SEQ ID NO: 1 or a subsequence thereof, as well as the amino acid sequence of SEQ ID NO: 2 or a fragment thereof, may be used to design a nucleic acid probe to identify and clone DNA encoding subtilases having proteolytic activity from strains of different genera or species according to methods well known in the art. In particular, such probes can be used for hybridisation with the genomic or cDNA of the genus or species of interest, following standard Southern blotting procedures, in order to identify and isolate the corresponding gene therein. Such probes can be considerably shorter than the entire sequence, but should be at least 15, preferably at least 25, and more preferably at least 35 nucleotides in length. Longer probes can also be used. Both DNA and RNA probes can be used. The probes are typically labeled for detecting the corresponding gene (for example, with 32P, 3H, 35S, biotin, or avidin). Such probes are encompassed by the present invention.

Thus, a genomic DNA or cDNA library prepared from such other organisms may be screened for DNA, which hybridizes with the probes described above and which encodes a subtilase according to the invention. Genomic or other DNA from such other organ-isms may be separated by agarose or polyacrylamide gel electrophoresis, or other separation techniques known by the skilled person. DNA from the libraries or the separated DNA may be transferred to and immobilized on nitrocellulose or other suit-able carrier materials. In order to identify a clone or DNA which is homologous with SEQ ID NO: 1 or a subsequence thereof, the carrier material is used in a Southern blot. For purposes of the present invention, hybridization indicates that the nucleic acid sequence hybridizes to a labelled nucleic acid probe corresponding to the nucleic acid sequence shown in SEQ ID NO: 1, its complementary strand, or a subsequence thereof, under low to high stringency conditions. Molecules to which the nucleic acid probe hybridizes under these conditions are detected using X-ray film.

For long probes of at least 100 nucleotides in length, low to high stringency conditions are defined as pre-hybridisation and hybridization at 42°C in 5X SSPE, 0.3% SDS, 200 □g/ml sheared and denatured salmon sperm DNA, and either 25% formamide for low stringency, 35% formamide for medium stringency, or 50% formamide for high stringency, following standard Southern blotting procedures.

For long probes of at least 100 nucleotides in length, the carrier material is finally washed three times each for 15 minutes using 2 x SSC, 0.2% SDS preferably at least at 50°C (low stringency), more preferably at least at 55°C (medium stringency), even more preferably at least at 65°C (high stringency).

For short probes, which are about 15 nucleotides to about 70 nucleotides in length, stringency conditions are defined as pre-hybridisation, hybridisation, and washing post-hybridisation at 5°C to 10°C below the calculated Tm using the calculation according to Bolton and McCarthy (1962, Proceedings of the National Academy of Sciences USA 48:1390) in 0.9 M NaCl, 0.09 M Tris-HCl pH 7.6, 6 mM EDTA, 0.5% NP-40, 1X Denhardt's solution, 1 mM sodium pyrophosphate, 1 mM sodium monobasic phosphate, 0.1 mM ATP, and 0.2 mg of yeast RNA per ml, following standard Southern blotting procedures.

For short probes, which are about 15 nucleotides to about 70 nucleotides in length, the carrier material is washed once in 6X SCC plus 0.1% SDS for 15 minutes and twice each for 15 minutes using 6X SSC at 5°C to 10°C below the calculated Tm.

It is well-known in the art that a so-called conservative substitution of one amino acid residue to a similar amino acid residue is expected to produce only a minor change in the characteristic of the enzyme.

Table I below lists groups of conservative amino acid substitutions.

**Table I**

| Conservative amino acid substitutions | |
|---|---|
| Common Property | Amino Acid |
| Basic (positive charge) | R = arginine |
| | K = lysine |
| | H = histidine |
| Acidic (negative charge) | E = glutamic acid |
| | D = aspartic acid |
| Polar | Q = glutamine |
| | N = asparagine |
| Hydrophobic | L = leucine |
| | I = isoleucine |
| | V = valine |
| | M = methionines |
| Aromatic | F = phenylalanine |
| | W = tryptophane |
| | Y = tyrosine |
| Small | G = glycine |
| | A = alanine |
| | S = serine |
| | T = threonine |

Therefore, in a further interesting embodiment of the invention, the protease having the amino acid sequence of SEQ ID NO: 2 is combined with a substitution, deletion and/or insertion of one or more amino acid residues.

Moreover, isolated proteases, preferably in a purified form, having immunochemical identity or partial immunochemical identity to the protease having the amino acid sequence of SEQ ID NO: 2, are also considered as being within the scope of the present invention. The immunochemical properties are determined by immunological cross-reaction identity tests by the well-known Ouchterlony double immunodiffusion procedure. Specifically, an antiserum containing polyclonal antibodies which are immunoreactive or bind to epitopes of the protease having the amino acid sequence of SEQ ID NO: 2 are prepared by immuniz-ing rabbits (or other rodents) according to the procedure described by Harboe and Ingild, In N.H. Axelsen, J. Krøll, and B. Weeks, editors, A Manual of Quantitative Immunoelectrophore-sis, Blackwell Scientific Publications, 1973, Chapter 23, or Johnstone and Thorpe, Immuno-chemistry in Practice, Blackwell Scientific Publications, 1982 (more specifically pages 27-31). A protease having immunochemical identity is a protease, which reacts with the antiserum in an identical fashion such as total fusion of precipitates, identical precipitate morphology, and/or identical electrophoretic mobility using a specific immunochemical technique. Axelsen, Bock, and Krøll describe a further explanation of immunochemical identity in N.H. Axelsen, J. Krøll, and B. Weeks, editors, A Manual of Quantitative Immunoelectrophoresis, Blackwell Sci-entific Publications, 1973, Chapter 10. A protease having partial immunochemical identity is a protease, which reacts with the antiserum in a partially identical fashion such as partial fusion of precipitates, partially identical precipitate morphology, and/or partially identical electrophoretic mobility using a specific immunochemical technique. Bock and Axelsen describe partial immunochemical identity in N.H. Axelsen, J. Krøll, and B. Weeks, editors, A Manual of Quantitative Immunoelectrophoresis, Blackwell Scientific Publications, 1973, Chapter 11.

The antibody may also be a monoclonal antibody. Monoclonal antibodies may be prepared and used, e.g. according to the methods of E. Harlow and D. Lane, editors, 1988, Antibodies, A Laboratory Manual, Cold Spring Harbor Press, Cold Spring Harbor, New York.

The present inventors isolated the gene encoding the subtilase having the amino acid sequence shown in SEQ ID NO: 2 and inserted it into Escherichia coli NN049696. The Escherichia coli NN049696 strain harbouring the gene was deposited according to the Budapest Treaty on the International Recognition of the Deposits of Microorganisms for the Purpose of Patent Procedures on 8 February 2000 at the Deutsche Sammlung von Mikroorganismen und Zellkulturen GmbH, Mascheroder Weg 1 B, D-38124 Braunschweig, Germany, and designated the accession No. DSM 15940.

In an interesting embodiment of the invention, the protease has more than 80.0%, or more than 85.0%, or more than 90.0%, or more than 92.0%, or more than 94.0%, or more than 96.0%, or more than 97.0%, or more than 98.0%, or more than 99.0% identity with the protease encoded by the protease encoding part of the polynucleotide cloned into a plasmid fragment present in Escherichia coli NN049696 deposited under the accession No. DSM 15940.

As mentioned above, the protease of the invention exhibits improved stability in detergent. Therefore, in order to enable the skilled person to select effective and preferred proteases for this purpose, the present inventors have provided a suitable test, which can easily be carried out by the skilled person in order to assess the performance of the protease in question.

Thus, the stability test disclosed in Example V herein may be employed to assess the stability of the selected protease. In other words, the example may be employed to assess the stability of a protease, when incorporated in a standard detergent composition, as compared to a reference system (incorporated in the same model detergent system and tested under identical conditions).

In an interesting aspect of the invention the protease - when tested in "Example V Stability in detergent" - has a residual activity of at least 40 % at 30°C, at least 50% at 30°C, such as at least 60% at 30°C, more preferably at least 70% at 30°C.

In another interesting aspect of the invention the protease - when tested in "Example V Stability in detergent" - has a residual activity of at least 50% at 35°C, such as at least 60% at 35°C, more preferably at least 70% at 35°C.

Therefore, proteases which are particular interesting for laundry wash purposes are such proteases which when tested in a model detergent composition comprising:

| | |
|---|---|
| Sulfonates | 20-30% |
| Nonionics | 0-4% |
| Na₂SO₄ | 2-8% |
| Na₂CO₃ | 20-40% |
| Na₂O*2SiO₂ | 1-3% |
| Zeolite 4A | 5-15% |
| Non polar hydrocarbons | 0-0.5% |
| Sodiumcitrate | 2-8% |
| PCA copolymer | 0-2% |

as described in the "Stability Test" herein, shows an improved stability as compared to a reference enzyme tested under identical conditions.

The protease of the invention may be constructed by standard techniques for artificial creation of diversity, such as by DNA shuffling of different protease genes (see WO 95/22625 and J.E. Ness et al., Nature Biotechnology, 17, 893-896 (1999)).

Obviously, the protease of the invention may also be isolated from a natural source, i.e. the protease of the invention may, for example, be a fungal polypeptide, and more preferably a filamentous fungal protease such as a Fusarium, Acremonium, Aspergillus, Aureobasidium, Cryptococcus, Filibasidium, Humicola, Magnaporthe, Mucor, Myceliophthora, Neocallimastix, Neurospora, Paecilomyces, Penicillium, Piromyces, Schizophyllum, Talaromyces, Thermoascus, Thielavia, Tolypocladium, or Trichoderma protease; or a..yeast protease such as a Candida, Kluyveromyces, Pichia, Saccharomyces, Schizosac-charomyces, or Yarrowia protease;

In another interesting embodiment, the protease is a Fusarium bactridioides, Fusarium cerealis, Fusarium crookwellense, Fusa-rium culmorum, Fusarium graminearum, Fusarium graminum, Fusarium heterosporum, Fusa-rium negundi, Fusarium oxysporum, Fusarium reticulatum, Fusarium roseum, Fusarium sam-bucinum, Fusarium sarcochroum, Fusarium sporotrichioides, Fusarium sulphureum, Fusarium torulosum, Fusarium trichothecioides, Fusarium venenatum, Aspergillus aculeatus, Aspergillus awamori, Aspergillus foetidus, Aspergillus japonicus, Aspergillus nidulans, Aspergillus niger, Aspergillus oryzae, Humicola insolens, Humicola la-nuginosa, Mucor miehei, Myceliophthora thermophila, Neurospora crassa, Penicillium pur-purogenum, Trichoderma harzianum, Trichoderma koningii, Trichoderma longibrachiatum, Trichoderma reesei, or Trichoderma viride protease.

In an interesting embodiment, the protease is a Saccharomyces carlsbergensis, Saccharomy-ces cerevisiae, Saccharomyces diastaticus, Saccharomyces douglasii, Saccharomyces kluy-veri, Saccharomyces norbensis or Saccharomyces oviformis protease.

It will be understood that for the aforementioned species, the invention encompasses both the perfect and imperfect states, and other taxonomic equivalents, e.g. anamorphs, regardless of the species name by which they are known. Those skilled in the art will readily recognize the identity of appropriate equivalents.

The protease of the present invention may also be a bacterial protease, e.g. a gram positive bacterial protease such as a Bacillus polypeptide, e.g., a Bacillus alkalophilus, Bacillus amyloliquefaciens, Bacillus brevis, Bacillus circulans, Bacillus coagulans, Bacillus lautus, Bacillus lentus, Bacillus licheniformis, Bacillus megaterium, Bacillus stearothermophilus, Bacillus subtilis, or Bacillus thuringiensis protease; or a Streptomyces protease, e.g., a Streptomyces lividans or Streptomyces murinus protease; or a gram negative bacterial protease, e.g. an E. coli or a Pseudomonas sp. Protease.

Strains of these species are readily accessible to the public in a number of culture collections, such as the American Type Culture Collection (ATCC), Deutsche Sammlung von Mikroorgan-ismen und Zellkulturen GmbH (DSM), Centraalbureau Voor Schimmelcultures (CBS), and Ag-ricultural Research Service Patent Culture Collection, Northern Regional Research Center (NRRL).

Furthermore, such proteases may be identified and obtained from other sources including microorganisms isolated from nature (e.g., soil, composts, water, etc.) using the above-mentioned probes. Techniques for isolating microorganisms from natural habitats are well known in the art. Similarly screening a genomic or cDNA library of another microorganism may then derive the polynucleotide. Once a polynucleotide encoding a protease has been detected with the probe(s), the sequence may be isolated or cloned by utilizing techniques which are known to those of ordinary skill in the art (see, e.g., Sambrook et al., 1989, supra).

In general standard procedures for cloning of genes and introducing insertions (random and/or site directed) into said genes may be used in order to obtain a protease enzyme of the inven-tion. For further description of suitable techniques reference is made to Examples herein (vide infra) and (Sambrook et al. (1989) Molecular cloning: A laboratory manual, Cold Spring Harbor lab., Cold Spring Harbor, NY; Ausubel, F. M. et al. (eds.) "Current protocols in Molecular Biol-ogy". John Wiley and Sons, 1995; Harwood, C. R., and Cutting, S. M. (eds.) "Molecular Bio-logical Methods for Bacillus". John Wiley and Sons, 1990); and WO 96/34946.

Further, a protease enzyme of the invention may be constructed by standard techniques for artificial creation of diversity, such as by DNA shuffling of different protease genes (WO 95/22625; Stemmer WPC, Nature 370:389-91 (1994)).

### POLYNUCLEOTIDES

The present invention also relates to an isolated polynucleotide, which encodes a protease of the present invention.

In one interesting embodiment, the polynucleotide has at least 86%, such as at least 87%, e.g. at least 88%, preferably at least 89%, such as at least 90%, e.g. at least 91 %, more preferably at least 92%, such as at least 93%, e.g. at least 94%, most preferably at least 95%, such as at least 96%, e.g. at least 97%, in particular at least 98%, preferably at least 99% identity with the polynucleotide shown as nucleotides 52 to 804 of SEQ ID NO: 1. In another interesting embodiment of the invention, the polynucleotide comprises the polynucleotide shown as nucleotides 127 to 804 of SEQ ID NO: 1, an allelic variant thereof, or a fragment thereof capable of encoding proteases according to the invention. Obviously, the polynucleotide may consist of the polynucleotide shown as nucleotides 127 to 804 of SEQ ID NO: 1.

The present invention also encompasses polynucleotides that encode a polypeptide having the amino acid sequence of SEQ ID NO: 2, which differ from SEQ ID NO: 2 by virtue of the de-generacy of the genetic code. The present invention also relates to subsequences of SEQ ID NO: 1 that encode fragments of SEQ ID NO: 2 that have proteolytic activity.

A subsequence of SEQ ID NO: 1 is a polynucleotide encompassed by nucleotides 52 to 804 SEQ ID NO: 1 except that one or more nucleotides from the 5' and/or 3' end have been deleted.

The techniques used to isolate or clone a polynucleotide encoding a polypeptide are known in the art and include isolation from genomic DNA, preparation from cDNA, or a combination thereof. The cloning of the polynucleotides of the present invention from such genomic DNA can be effected, e.g., by using the well-known polymerase chain reaction (PCR) or antibody screening of expression libraries to detect cloned DNA fragments with shared structural fea-tures. See, e.g., Innis et al., 1990, PCR: A Guide to Methods and Application, Academic Press, New York. Other nucleic acid amplification procedures such as ligase chain reaction (LCR), ligated activated transcription (LAT) and nucleic acid sequence-based amplification (NASBA) may be used.

An isolated polynucleotide can for example be obtained by standard cloning procedures used in genetic engineering to relocate the polynucleotide from its natural location to a different site where it will be reproduced. The cloning procedures may involve excision and isolation of a desired nucleic acid fragment comprising the polynucleotide encoding the protease, insertion of the fragment into a vector molecule, and incorporation of the recombinant vector into a host cell where multiple copies or clones of the polynucleotide will be replicated. The polynucleotide may be of genomic, cDNA, RNA, semi-synthetic, synthetic origin, or any combinations thereof.

For purposes of the present invention, the degree of identity between two polynucleotides is determined is described above.

Modification of a polynucleotide encoding a protease of the present invention may be necessary for the synthesis of proteases substantially similar to the protease. The term "substantially similar" to the protease refers to non-naturally occurring forms of the protease. These proteases may differ in some engineered way from the protease isolated from its native source, e.g., variants that differ in specific activity, thermo stability, pH optimum, or the like. The variant sequence may be constructed on the basis of the polynucleotide presented as the polypeptide encoding part of SEQ ID NO: 1, e.g., a subsequence thereof, and/or by introduction of nucleotide substitutions which do not give rise to another amino acid sequence of the protease encoded by the nucleic acid sequence, but which correspond to the codon usage of the host organism intended for production of the enzyme, or by introduction of nucleotide substitutions which may give rise to a different amino acid sequence. For a general description of nucleotide substitution see, e.g., Ford et al., 1991, Protein Expression and Purification 2: 95-107.

It will be apparent to those skilled in the art that such substitutions can be made outside the regions critical to the function of the molecule and still result in an active protease. Amino acid residues essential to the activity of the polypeptide encoded by the isolated polynucleotide of the invention, and therefore preferably not subject to substitution, may be identified according to procedures known in the art, such as site-directed mutagenesis or alanine-scanning mutagenesis (see, e.g., Cunningham and Wells, 1989, Science 244: 1081-1085). In the latter technique, mutations are introduced at every positively charged residue in the molecule, and the resultant mutant molecules are tested for proteolytic activity to identify amino acid residues that are critical to the activity of the molecule. Sites of substrate-enzyme interaction can also be determined by analysis of the three-dimensional structure as determined by such techniques as nuclear magnetic resonance analysis, crystallography or photo affinity labelling (see, e.g., de Vos et al., 1992, Science 255: 306-312; Smith et al., 1992, Journal of Molecular Biology 224: 899-904; Wlodaver et al., 1992, FEBS Letters 309: 59-64).

### NUCLEIC ACID CONSTRUCTS

The present invention also relates to nucleic acid constructs comprising a polynucleotide of the present invention operably linked to one or more control sequences capable of directing the expression of the polypeptide in a suitable host cell.

An isolated polynucleotide encoding a protease of the present invention may be manipulated in a variety of ways to provide for expression of the protease. Manipulation of the polynucleotide prior to its insertion into a vector may be desirable or necessary depending on the expression vector. The techniques for modifying polynucleotides utilising recombinant DNA methods are well-known in the art.

The control sequences include all components that are necessary or advantageous for the expression of a protease of the present invention. Each control sequence may be native or foreign to the polynucleotide encoding the protease. Such control sequences include, but are not limited to, a leader, polyadenylation sequence, propeptide sequence, promoter, signal peptide sequence, and transcription terminator. At a minimum, the control sequences include a promoter, and transcriptional and translational stop signals. The control sequences may be provided with linkers for the purpose of introducing specific restriction sites facilitating ligation of the control sequences with the coding region of the polynucleotide encoding a protease.

The control sequence may be an appropriate promoter sequence, a polynucleotide that is recognized by a host cell for expression of the nucleic acid sequence. The promoter sequence contains transcriptional control sequences that mediate the expression of the protease. The promoter may be any polynucleotide that shows transcriptional activity in the host cell of choice including mutant, truncated, and hybrid promoters, and may be obtained from genes encoding extracellular or intracellular proteases either homologous or heterologous to the host cell.

Examples of suitable promoters for directing the transcription of the nucleic acid constructs of the present invention in a filamentous fungal host cell are promoters obtained from the genes for Aspergillus oryzae TAKA amylase, Rhizomucor miehei aspartic proteinase, Aspergillus ni-ger neutral alpha-amylase, Aspergillus niger acid stable alpha-amylase, Aspergillus niger or Aspergillus awamori glucoamylase (glaA), Rhizomucor miehei lipase, Aspergillus oryzae alka-line protease, Aspergillus oryzae triose phosphate isomerase, Aspergillus nidulans acetami-dase, and Fusarium oxysporum trypsin-like protease (WO 96/00787), as well as the NA2-tpi promoter (a hybrid of the promoters from the genes for Aspergillus niger neutral alpha-amylase and Aspergillus oryzae triose phosphate isomerase), and mutant, truncated, and hy-brid promoters thereof.

In a yeast host, useful promoters are obtained from the genes for Saccharomyces cerevisiae enolase (ENO-1), Saccharomyces cerevisiae galactokinase (GAL1), Saccharomyces cere-visiae alcohol dehydrogenase/glyceraldehyde-3-phosphate dehydrogenase (ADH2/GAP), and Saccharomyces cerevisiae 3-phosphoglycerate kinase. Other useful promoters for yeast host cells are described by Romanos et al., 1992, Yeast 8: 423-488.

Examples of suitable promoters for directing the transcription of the nucleic acid constructs of the present invention, especially in a bacterial host cell, are the promoters obtained from the E. coli lac operon, Streptomyces coelicolor agarase gene (dagA), Bacillus subtilis levansu-crase gene (sacB), Bacillus licheniformis alpha-amylase gene (amyL), Bacillus stearothermo-philus maltogenic amylase gene (amyM), Bacillus amyloliquefaciens alpha-amylase gene (amyQ), Bacillus licheniformis penicillinase gene (penP), Bacillus subtilis xylA and xylB genes, and prokaryotic beta-lactamase gene (Villa-Kamaroff et al., 1978, Proceedings of the National Academy of Sciences USA 75: 3727-3731), as well as the tac promoter (DeBoer et al., 1983, Proceedings of the National Academy of Sciences USA 80: 21-25). Further promoters are de-scribed in "Useful proteins from recombinant bacteria" in Scientific American, 1980, 242: 74-94; and in Sambrook et al., 1989, supra.

The control sequence may also be a suitable transcription terminator sequence, a sequence recognized by a host cell to terminate transcription. The terminator sequence is operably linked to the 3' terminus of the polynucleotide encoding the protease. Any terminator that is functional in the host cell of choice may be used in the present invention.

Preferred terminators for filamentous fungal host cells are obtained from the genes for Asper-gillus oryzae TAKA amylase, Aspergillus niger glucoamylase, Aspergillus nidulans anthranilate synthase, Aspergillus niger alpha-glucosidase, and Fusarium oxysporum trypsin-like protease.

Preferred terminators for yeast host cells are obtained from the genes for Saccharomyces cerevisiae enolase, Saccharomyces cerevisiae cytochrome C (CYC1), and Saccharomyces cerevisiae glyceraldehyde-3-phosphate dehydrogenase. Romanos et al., 1992, supra, de-scribe other useful terminators for yeast host cells.

The control sequence may also be a suitable leader sequence, a non-translated region of an mRNA that is important for translation by the host cell. The leader sequence is operably linked to the 5' terminus of the polynucleotide encoding the polypeptide. Any leader sequence that is functional in the host cell of choice may be used in the present invention.

Preferred leaders for filamentous fungal host cells are obtained from the genes for Aspergillus oryzae TAKA amylase and Aspergillus nidulans triose phosphate isomerase.

Suitable leaders for yeast host cells are obtained from the genes for Saccharomyces cere-visiae enolase (ENO-1), Saccharomyces cerevisiae 3-phosphoglycerate kinase, Saccharomy-ces cerevisiae alpha-factor, and Saccharomyces cerevisiae alcohol dehydrogenase/glyceraldehyde-3-phosphate dehydrogenase (ADH2/GAP).

The control sequence may also be a polyadenylation sequence, a sequence operably linked to the 3' terminus of the polynucleotide and which, when transcribed, is recognized by the host cell as a signal to add polyadenosine residues to transcribed mRNA. Any polyadenylation se-quence that is functional in the host cell of choice may be used in the present invention.

Preferred polyadenylation sequences for filamentous fungal host cells are obtained from the genes for Aspergillus oryzae TAKA amylase, Aspergillus niger glucoamylase, Aspergillus nidu-lans anthranilate synthase, Fusarium oxysporum trypsin-like protease, and Aspergillus niger alpha-glucosidase.

Useful polyadenylation sequences for yeast host cells are described by Guo and Sherman, 1995, Molecular Cellular Biology 15: 5983-5990.

The control sequence may also be a signal peptide coding region that codes for an amino acid sequence linked to the amino terminus of a protease and directs the encoded protease into the cell's secretory pathway. The 5' end of the coding sequence of the polynucleotide may inherently contain a signal peptide coding region naturally linked in translation reading frame with the segment of the coding region that encodes the secreted protease. Alternatively, the 5' end of the coding sequence may contain a signal peptide coding region that is foreign to the coding sequence. The foreign signal peptide coding region may be required where the coding sequence does not naturally contain a signal peptide coding region. Alternatively, the foreign signal peptide coding region may simply replace the natural signal peptide coding region in order to enhance secretion of the protease. However, any signal peptide coding region that directs the expressed protease into the secretory pathway of a host cell of choice may be used in the present invention.

Effective signal peptide coding regions for filamentous fungal host cells are the signal peptide coding regions obtained from the genes for Aspergillus oryzae TAKA amylase, Aspergillus ni-ger neutral amylase, Aspergillus niger glucoamylase, Rhizomucor miehei aspartic proteinase, Humicola insolens cellulase, and Humicola lanuginosa lipase.

Useful signal peptides for yeast host cells are obtained from the genes for Saccharomyces cerevisiae alpha-factor and Saccharomyces cerevisiae invertase. Romanos et al., 1992, su-pra, describe other useful signal peptide coding regions.

Effective signal peptide coding regions for bacterial host cells are the signal peptide coding regions obtained from the genes for Bacillus NCIB 11837 maltogenic amylase, Bacillus stearothermophilus alpha-amylase, Bacillus licheniformis subtilisin, Bacillus licheniformis beta-lactamase, Bacillus stearothermophilus neutral proteases (nprT, nprS, nprM), and Bacillus subtilis prsA. Further signal peptides are described by Simonen and Palva, 1993, Microbiological Reviews 57: 109-137.

The control sequence may also be a propeptide coding region that codes for an amino acid sequence positioned at the amino terminus of a protease. The resultant polypeptide is known as a proenzyme or propolypeptide (or a zymogen in some cases). A propolypeptide is gener-ally inactive and can be converted to a mature active polypeptide by catalytic or autocatalytic cleavage of the propeptide from the propolypeptide. The propeptide coding region may be ob-tained from the genes for Bacillus subtilis alkaline protease (aprE), Bacillus subtilis neutral pro-tease (nprT), Saccharomyces cerevisiae alpha-factor, Rhizomucor miehei aspartic proteinase, and Myceliophthora thermophila laccase (WO 95/33836).

Where both signal peptide and propeptide regions are present at the amino terminus of a sub-tilase, the propeptide region is positioned next to the amino terminus of a protease and the signal peptide region is positioned next to the amino terminus of the propeptide region.

It may also be desirable to add regulatory sequences that allow the regulation of the expres-sion of the polypeptide relative to the growth of the host cell. Examples of regulatory systems are those which cause the expression of the gene to be turned on or off in response to a chemical or physical stimulus, including the presence of a regulatory compound. Regulatory systems in prokaryotic systems include the lac, tac, and trp operator systems. In yeast, the ADH2 system or GAL1 system may be used. In filamentous fungi, the TAKA alpha-amylase promoter, Aspergillus niger glucoamylase promoter, and Aspergillus oryzae glucoamylase promoter may be used as regulatory sequences. Other examples of regulatory sequences are those that allow for gene amplification. In eukaryotic systems, these include the dihydrofolate reductase gene that is amplified in the presence of methotrexate, and the metallothionein genes that are amplified with heavy metals. In these cases, the polynucleotide encoding the polypeptide would be operably linked with the regulatory sequence.

### EXPRESSION VECTORS

The present invention also relates to a recombinant expression vector comprising the nucleic acid construct of the invention, a promoter, and transcriptional and translational stop signals.

The recombinant expression vector comprising the nucleic acid construct encoding the enzyme of the invention may be any vector that may conveniently be subjected to recombinant DNA procedures.

The choice of vector will often depend on the host cell into which it is to be introduced. Thus, the vector may be an autonomously replicating vector, i.e. a vector that exists as an extrachromosomal entity, the replication of which is independent of chromosomal replication, e.g. a plasmid. Alternatively, the vector may be one that on introduction into a host cell is integrated into the host cell genome in part or in its entirety and replicated together with the chromosome(s) into which it has been integrated.

The vector is preferably an expression vector in which the DNA sequence encoding the enzyme of the invention is operably linked to additional segments required for transcription of the DNA. In general, the expression vector is derived from plasmid or viral DNA, or may contain elements of both. The term "operably linked" indicates that the segments are arranged so that they function in concert for their intended purposes, e.g. transcription initiates in a promoter and proceeds through the DNA sequence coding for the enzyme.

The promoter may be any DNA sequence that shows transcriptional activity in the host cell of choice and may be derived from genes encoding proteins either homologous or heterologous to the host cell.

Examples of suitable promoters for use in bacterial host cells include the promoter of the Bacillus stearothermophilus maltogenic amylase gene, the Bacillus licheniformis alpha-amylase gene, the Bacillus amyloliquefaciens alpha-amylase gene, the Bacillus subtilis alkaline protease gene, or the Bacillus pumilus xylosidase gene, or the phage Lambda PR or PL promoters or the E. coli lac, trp or tac promoters.

The DNA sequence encoding the enzyme of the invention may also, if necessary, be operably connected to a suitable terminator.

The recombinant vector of the invention may further comprise a DNA sequence enabling the vector to replicate in the host cell in question.

The vector may also comprise a selectable marker, e.g. a gene the product of which complements a defect in the host cell, or a gene encoding resistance to e.g. antibiotics like kanamycin, chloramphenicol, erythromycin, tetracycline, spectinomycine, or the like, or resistance to heavy metals or herbicides.

To direct an enzyme of the present invention into the secretory pathway of the host cells, a secretory signal sequence (also known as a leader sequence, prepro sequence or pre sequence) may be provided in the recombinant vector. The secretory signal sequence is joined to the DNA sequence encoding the enzyme in the correct reading frame. Secretory signal sequences are commonly positioned 5' to the DNA sequence encoding the enzyme. The secretory signal sequence may be that normally associated with the enzyme or may be from a gene encoding another secreted protein.

The procedures used to ligate the DNA sequences coding for the present enzyme, the promoter and optionally the terminator and/or secretory signal sequence, respectively, or to assemble these sequences by suitable PCR amplification schemes, and to insert them into suitable vectors containing the information necessary for replication or integration, are well known to persons skilled in the art (cf., for instance, Sambrook et al., op.cit.).

### HOST CELL

The present invention also relates to a recombinant host cell comprising the nucleic acid construct of the invention.

The DNA sequence encoding the present enzyme introduced into the host cell may be either homologous or heterologous to the host in question. If homologous to the host cell, i.e. produced by the host cell in nature, it will typically be operably connected to another promoter sequence or, if applicable, another secretory signal sequence and/or terminator sequence than in its natural environment. The term "homologous" is intended to include a DNA sequence encoding an enzyme native to the host organism in question. The term "heterologous" is intended to include a DNA sequence not expressed by the host cell in nature. Thus, the DNA sequence may be from another organism, or it may be a synthetic sequence.

The host cell into which the DNA construct or the recombinant vector of the invention is introduced may be any cell that is capable of producing the present enzyme and includes bacteria, yeast, fungi and higher eukaryotic cells including plants.

Examples of bacterial host cells which on cultivation are capable of producing the enzyme of the invention are gram-positive bacteria such as strains of Bacillus, such as strains of B. subtilis, B. licheniformis, B. lentus, B. brevis, B. stearothermophilus, B. alkalophilus, B. amyloliquefaciens, B. coagulans, B. circulans, B. lautus, B. megaterium or B. thuringiensis, in particular B. lentus, or strains of Streptomyces, such as S. lividans or S. murinus, or gram-negative bacteria such as Escherichia coli.

The transformation of the bacteria may be effected by protoplast transformation, electroporation, conjugation, or by using competent cells in a manner known per se (cf. Sambrook et al., supra).

When expressing the enzyme in bacteria such as E. coli, the enzyme may be retained in the cytoplasm, typically as insoluble granules (known as inclusion bodies), or may be directed to the periplasmic space by a bacterial secretion sequence. In the former case, the cells are lysed and the granules are recovered and denatured after which the enzyme is refolded by diluting the denaturing agent. In the latter case, the enzyme may be recovered from the periplasmic space by disrupting the cells, e.g. by sonication or osmotic shock, to release the contents of the periplasmic space and recovering the enzyme.

When expressing the enzyme in gram-positive bacteria such as Bacillus or Streptomyces strains, the enzyme may be retained in the cytoplasm, or may be directed to the extracellular medium by a bacterial secretion sequence. In the latter case, the enzyme may be recovered from the medium as described below.

In another embodiment of the invention, the fungal host cell is a yeast cell. "Yeast" as used herein includes ascosporogenous yeast (Endomycetales), basidiosporogenous yeast, and yeast belonging to the Fungi Imperfecti (Blastomycetes). Since the classification of yeast may change in the future, for the purposes of this invention, yeast shall be defined as described in Biology and Activities of Yeast (Skinner, F.A., Passmore, S.M., and Davenport, R.R., eds., Soc. App. Bacteriol. Symposium Series No. 9, 1980).

In a preferred embodiment, the yeast host cell is a Candida, Hansenula, Kluyveromyces, Pichia, Saccharomyces, Schizosaccharomyces, or Yarrowia cell.

In a more preferred embodiment, the yeast host cell is a Saccharomyces carlsbergensis, Sac-charomyces cerevisiae, Saccharomyces diastaticus, Saccharomyces douglasii, Saccharomy-ces kluyveri, Saccharomyces norbensis or Saccharomyces oviformis cell. In another most preferred embodiment, the yeast host cell is a Kluyveromyces lactis cell. In another most pre-ferred embodiment, the yeast host cell is a Yarrowia lipolytica cell.

In another preferred embodiment, the fungal host cell is a filamentous fungal cell. "Filamen-tous fungi" include all filamentous forms of the subdivision Eumycota and Oomycota (as de-fined by Hawksworth et al., 1995, supra). The filamentous fungi are characterized by a myce-lial wall composed of chitin, cellulose, glucan, chitosan, mannan, and other complex polysac-charides. Vegetative growth is by hyphal elongation and carbon catabolism is obligately aero-bic. In contrast, vegetative growth by yeasts such as Saccharomyces cerevisiae is by budding of a unicellular thallus and carbon catabolism may be fermentative.

In an even more preferred embodiment, the filamentous fungal host cell is a cell of a species of, but not limited to, Fusarium, Acremonium, Aspergillus, Humicola, Mucor, Myceliophthora, Neurospora, Penicillium, Thielavia, Tolypocladium, or Trichoderma.

In a most preferred embodiment, the filamentous fungal host cell is an Aspergillus awamori, Aspergillus foetidus, Aspergillus japonicus, Aspergillus nidulans, Aspergillus niger or Aspergil-lus oryzae cell. In another most preferred embodiment, the filamentous fungal host cell is a Fusarium bactridioides, Fusarium cerealis, Fusarium crookwellense, Fusarium culmorum, Fusarium graminearum, Fusarium graminum, Fusarium heterosporum, Fusarium negundi, Fusarium oxysporum, Fusarium reticulatum, Fusarium roseum, Fusarium sambucinum, Fusa-rium sarcochroum, Fusarium sporotrichioides, Fusarium sulphureum, Fusarium torulosum, Fusarium trichothecioides, or Fusarium venenatum cell. In an even most preferred embodi-ment, the filamentous fungal parent cell is a Fusarium venenatum (Nirenberg sp. nov.) cell. In another most preferred embodiment, the filamentous fungal host cell is a Humicola insolens, Humicola lanuginosa, Mucor miehei, Myceliophthora thermophila, Neurospora crassa, Penicil-lium purpurogenum, Thielavia terrestris, Trichoderma harzianum, Trichoderma koningii, Trichoderma longibrachiatum, Trichoderma reesei, or Trichoderma viride cell.

Fungal cells may be transformed by a process involving protoplast formation, transformation of the protoplasts, and regeneration of the cell wall in a manner known per se. Suitable proce-dures for transformation of Aspergillus host cells are described in EP 238 023 and Yelton et al., 1984, Proceedings of the National Academy of Sciences USA 81: 1470-1474. Suitable methods for transforming Fusarium species are described by Malardier et al., 1989, Gene 78: 147-156 and WO 96/00787. Yeast may be transformed using the procedures described by Becker and Guarente, In Abelson, J.N. and Simon, M.I., editors, Guide to Yeast Genetics and Molecular Biology, Methods in Enzymology, Volume 194, pp 182-187, Academic Press, Inc., New York; Ito et al., 1983, Journal of Bacteriology 153: 163; and Hinnen et al., 1978, Proceed-ings of the National Academy of Sciences USA 75: 1920.

### METHOD OF PRODUCING A PROTEASE OF THE INVENTION

The present invention further relates to a method for producing a protease of the invention, the method comprising:
a) cultivating a recombinant host cell of the invention under conditions conducive to the production of the protease; and
b) recovering the protease.

When an expression vector comprising a DNA sequence encoding the enzyme is transformed into a heterologous host cell, it is possible to enable heterologous recombinant production of the enzyme of the invention.

Thereby it is possible to make a highly purified protease composition, characterised in being free from homologous impurities.

In this context, homologous impurities mean any impurities (e.g. other polypeptides than the enzyme of the invention) that originate from the homologous cell where the enzyme of the invention is originally obtained from.

The medium used to culture the transformed host cells may be any conventional medium suitable for growing the host cells in question. The expressed protease may conveniently be secreted into the culture medium and may be recovered there from by well-known procedures including separating the cells from the medium by centrifugation or filtration, precipitating proteinaceous components of the medium by means of a salt such as ammonium sulphate, followed by chromatographic procedures such as ion exchange chromatography, affinity chromatography or the like.

### USE OF A PROTEASE OF THE INVENTION

A protease enzyme of the invention may be used for a number of industrial applications, in particular within the detergent industry. Thus, the present invention also relates to a cleaning or detergent composition, preferably a laundry or dish washing composition, comprising the protease enzyme of the invention.

### DETERGENT COMPOSITIONS COMPRISING THE PROTEASE OF THE INVENTION

In general, cleaning and detergent compositions are well described in the art and reference is made to WO 96/34946; WO 97/07202; WO 95/30011 for further description of suitable cleaning and detergent compositions.

Furthermore, the examples herein demonstrate the improvements in stability in detergent for the proteases of the invention.

### Detergent Compositions

The enzyme of the invention may be added to and thus become a component of a cleaning or detergent composition.

The detergent composition of the invention may for example be formulated as a hand or ma-chine laundry detergent composition including a laundry additive composition suitable for pre-treatment of stained fabrics and a rinse added fabric softener composition, or be formulated as a detergent composition for use in general household hard surface cleaning operations, or be formulated for hand or machine dishwashing operations.

In a specific aspect, the invention provides a detergent additive comprising the protease of the invention. The detergent additive as well as the detergent composition may comprise one or more other enzymes such as another protease, a lipase, a cutinase, an amylase, a carbohydrase, a cellulase, a pectinase, a mannanase, an arabinase, a galactanase, a xylanase, an oxidase, e.g., a laccase, and/or a peroxidase.

In general the properties of the chosen enzyme(s) should be compatible with the selected de-tergent, (i.e. pH-optimum, compatibility with other enzymatic and non-enzymatic ingredients, etc.), and the enzyme(s) should be present in effective amounts.

Proteases: Suitable proteases include those of animal, vegetable or microbial origin. Microbial origin is preferred. Chemically modified or protein engineered mutants are included. The protease may be a serine protease or a metallo protease, preferably an alkaline microbial protease or a trypsin-like protease. Examples of alkaline proteases are subtilisins, especially those derived from Bacillus, e.g., subtilisin Novo, subtilisin Carlsberg, subtilisin 309, subtilisin 147 and subtilisin 168 (described in WO 89/06279). Examples of trypsin-like proteases are trypsin (e.g. of porcine or bovine origin) and the Fusarium protease described in WO 89/06270 and WO 94/25583.

Examples of useful proteases are the variants described in WO 92/19729, WO 98/20115, WO 98/20116, and WO 98/34946, especially the variants with substitutions in one or more of the following positions: 27, 36, 56, 76, 87, 95, 96, 97, 98, 99, 100, 101, 102, 103, 104, 120, 123, 159, 167, 170, 206, 218, 222, 224, 232, 235, 236, 245, 248, 252 and 274 (BPN' numbering).

Preferred commercially available protease enzymes include Alcalase™, Savinase™, Primase™, Duralase™, Esperase™, and Kannase™ (Novozymes A/S), Maxatase™, Maxacal™, Maxapem™, Properase™, Purafect™, Purafect OxP™, FN2™, and FN3™ (Genencor International Inc.).

Lipases: Suitable lipases include those of bacterial or fungal origin. Chemically modified or protein engineered mutants are included. Examples of useful lipases include lipases from Humicola (synonym Thermomyces), e.g. from H. lanuginosa (T. lanuginosus) as described in EP 258 068 and EP 305 216 or from H. insolens as described in WO 96/13580, a Pseudomonas lipase, e.g. from P. alcaligenes or P. pseudoalcaligenes (EP 218 272), P. cepacia (EP 331 376), P. stutzeri (GB 1,372,034), P. fluorescens, Pseudomonas sp. strain SD 705 (WO 95/06720 and WO 96/27002), P. wisconsinensis (WO 96/12012), a Bacillus lipase, e.g. from B. subtilis (Dartois et al. (1993), Biochemica et Biophysica Acta, 1131, 253-360), B. stearothermophilus (JP 64/744992) or B. pumilus (WO 91/16422).

Other examples are lipase variants such as those described in WO 92/05249, WO 94/01541, EP 407 225, EP 260 105, WO 95/35381, WO 96/00292, WO 95/30744, WO 94/25578, WO 95/14783, WO 95/22615, WO 97/04079 and WO 97/07202.

Preferred commercially available lipase enzymes include Lipolase™ and Lipolase UItra™ (Novozymes A/S).

Amylases: Suitable amylases (alfa and/or beta) include those of bacterial or fungal origin. Chemically modified or protein engineered mutants are included. Amylases include, for example, alfa-amylases obtained from Bacillus, e.g. a special strain of B. licheniformis, described in more detail in GB 1,296,839.

Examples of useful amylases are the variants described in WO 94/02597, WO 94/18314, WO 96/23873, and WO 97/43424, especially the variants with substitutions in one or more of the following positions: 15, 23, 105, 106, 124, 128, 133, 154, 156, 181, 188, 190, 197, 202, 208, 209, 243, 264, 304, 305, 391, 408, and 444.

Commercially available amylases are Duramyl™, Termamyl™, Fungamyl™ and BAN™ (Novozymes A/S), Rapidase™ and Purastar™ (from Genencor International Inc.).

Cellulases: Suitable cellulases include those of bacterial or fungal origin. Chemically modified or protein engineered mutants are included. Suitable cellulases include cellulases from the genera Bacillus, Pseudomonas, Humicola, Fusarium, Thielavia, Acremonium, e.g. the fungal cellulases produced from Humicola insolens, Myceliophthora thermophila and Fusarium oxysporum disclosed in US 4,435,307, US 5,648,263, US 5,691,178, US 5,776,757 and WO 89/09259.

Especially suitable cellulases are the alkaline or neutral cellulases having colour care benefits. Examples of such cellulases are cellulases described in EP 0 495 257, EP 0 531 372, WO 96/11262, WO 96/29397, WO 98/08940. Other examples are cellulase variants such as those described in WO 94/07998, EP 0 531 315, US 5,457,046, US 5,686,593, US 5,763,254, WO 95/24471, WO 98/12307 and PCT/DK98/00299.

Commercially available cellulases include Celluzyme™, and Carezyme™ (Novozymes A/S), Clazinase™, and Puradax HA™ (Genencor International Inc.), and KAC-500(B) ™ (Kao Corporation).

Peroxidases/Oxidases: Suitable peroxidases/oxidases include those of plant, bacterial or fungal origin. Chemically modified or protein engineered mutants are included. Examples of useful peroxidases include peroxidases from Coprinus, e.g. from C. cinereus, and variants thereof as those described in WO 93/24618, WO 95/10602, and WO 98/15257.

The detergent enzyme(s) may be included in a detergent composition by adding separate ad-ditives containing one or more enzymes, or by adding a combined additive comprising all of these enzymes. A detergent additive of the invention, i.e. a separate additive or a combined additive, can be formulated e.g. as a granulate, a liquid, a slurry, etc. Preferred detergent addi-tive formulations are granulates, in particular non-dusting granulates, liquids, in particular sta-bilized liquids, or slurries.

Non-dusting granulates may be produced, e.g. as disclosed in US 4,106,991 and 4,661,452 and may optionally be coated by methods known in the art. Examples of waxy coating materi-als are poly(ethylene oxide) products (polyethylene glycol, PEG) with mean molar weights of 1000 to 20000; ethoxylated nonylphenols having from 16 to 50 ethylene oxide units; ethoxy-lated fatty alcohols in which the alcohol contains from 12 to 20 carbon atoms and in which there are 15 to 80 ethylene oxide units; fatty alcohols; fatty acids; and mono- and di- and triglycerides of fatty acids. Examples of film-forming coating materials suitable for application by fluid bed techniques are given in GB 1483591. Liquid enzyme preparations may, for instance, be stabilized by adding a polyol such as propylene glycol, a sugar or sugar alcohol, lactic acid or boric acid according to established methods. Protected enzymes may be pre-pared according to the method disclosed in EP 238,216.

The detergent composition of the invention may be in any convenient form, e.g. a bar, a tablet, a powder, a granule, a paste or a liquid. A liquid detergent may be aqueous, typically containing up to 70% water and 0-30% organic solvent, or non-aqueous.

The detergent composition typically comprises one or more surfactants, which may be non-ionic including semi-polar and/or anionic and/or cationic and/or zwitterionic. The surfactants are typically present at a level of from 0.1% to 60% by weight.

When included therein the detergent will usually contain from about 1% to about 40% of an anionic surfactant such as linear alkylbenzenesulfonate, alpha-olefinsulfonate, alkyl sulfate (fatty alcohol sulfate), alcohol ethoxysulfate, secondary alkanesulfonate, alpha-sulfo fatty acid methyl ester, alkyl- or alkenylsuccinic acid or soap.

When included therein the detergent will usually contain from about 0.2% to about 40% of a non-ionic surfactant such as alcohol ethoxylate, nonylphenol ethoxylate, alkylpolyglycoside, alkyldimethylamineoxide, ethoxylated fatty acid monoethanolamide, fatty acid monoethanola-mide, polyhydroxy alkyl fatty acid amide, or N-acyl N-alkyl derivatives of glucosamine ("glucamides").

The detergent may contain 0-65% of a detergent builder or complexing agent such as zeolite, diphosphate, triphosphate, phosphonate, carbonate, citrate, nitrilotriacetic acid, ethylenedia-minetetraacetic acid, diethylenetriaminepentaacetic acid, alkyl- or alkenylsuccinic acid, soluble silicates or layered silicates (e.g. SKS-6 from Hoechst).

The detergent may comprise one or more polymers. Examples are carboxymethylcellulose, poly(vinylpyrrolidone), poly (ethylene glycol), poly(vinyl alcohol), poly(vinylpyridine-N-oxide), poly(vinylimidazole), polycarboxylates such as polyacrylates, maleic/acrylic acid copolymers and lauryl methacrylate/acrylic acid copolymers.

The detergent may contain a bleaching system that may comprise a hydrogen peroxide source such as perborate or percarbonate that may be combined with a peracid-forming bleach activator such as tetraacetylethylenediamine or nonanoyloxybenzenesulfonate. Alternatively, the bleaching system may comprise peroxyacids of e.g. the amide, imide, or sulfone type.

The enzyme(s) of the detergent composition of the invention may be stabilized using conven-tional stabilizing agents, e.g. a polyol such as propylene glycol or glycerol, a sugar or sugar alcohol, lactic acid, boric acid, or a boric acid derivative, e.g. an aromatic borate ester, or a phenyl boronic acid derivative such as 4-formylphenyl boronic acid, and the composition may be formulated as described in e.g. WO 92/19709 and WO 92/19708.

The detergent may also contain other conventional detergent ingredients such as e.g. fabric conditioners including clays, foam boosters, suds suppressors, anti-corrosion agents, soil-suspending agents, anti-soil redeposition agents, dyes, bactericides, optical brighteners, hy-drotropes, tarnish inhibitors, or perfumes.

It is at present contemplated that in the detergent compositions any enzyme, in particular the enzyme of the invention, may be added in an amount corresponding to 0.01-100 mg of en-zyme protein per litre of wash liquor, preferably 0.05-5 mg of enzyme protein per litre of wash liquor, in particular 0.1-1 mg of enzyme protein per litre of wash liquor.

The enzyme of the invention may additionally be incorporated in the detergent formulations disclosed in WO 97/07202.

The invention is described in further detail in the following examples that are not in any way intended to limit the scope of the invention as claimed.

In the detergent compositions, the abbreviated component identifications have the following meanings:
- LAS:: Sodium linear C₁₂ alkyl benzene sulfonate
- TAS:: Sodium tallow alkyl sulfate
- XYAS:: Sodium C_{1X} - C_{1Y} alkyl sulfate
- SS:: Secondary soap surfactant of formula 2-butyl octanoic acid
- 25EY:: A C₁₂-C₁₅ predominantly linear primary alcohol condensed with an average of Y moles of ethylene oxide
- 45EY:: A C₁₄-C₁₅ predominantly linear primary alcohol condensed with an average of Y moles of ethylene oxide
- XYEZS:: C_{1X}-C_{1Y} sodium alkyl sulfate condensed with an average of Z moles of ethylene oxide per mole
- Non-ionic:: C₁₃-C₁₅ mixed ethoxylated/propoxylated fatty alcohol with an average degree of ethoxylation of 3.8 and an average degree of propoxylation of 4.5 sold under the trade name Plurafax LF404 by BASF GmbH
- CFAA:: C₁₂-C₁₄ alkyl N-methyl glucamide
- TFAA:: C₁₆-C₁₈ alkyl N-methyl glucamide
- Silicate:: Amorphous Sodium Silicate (SiO₂:Na₂O ratio = 2.0)
- NaSKS-6:: Crystalline layered silicate of formula delta-Na₂Si₂O₅
- Carbonate:: Anhydrous sodium carbonate
- Phosphate:: Sodium tripolyphosphate
- MA/AA:: Copolymer of 1:4 maleic/acrylic acid, average molecular weight about 80,000
- Polyacrylate:: Polyacrylate homopolymer with an average molecular weight of 8,000 sold under the trade name PA30 by BASF GmbH
- Zeolite A:: Hydrated Sodium Aluminosilicate of formula Na₁₂(AlO₂SiO₂)₁₂.27H₂O having a primary particle size in the range from 1 to 10 micrometers
- Citrate:: Tri-sodium citrate dihydrate
- Citric:: Citric Acid
- Perborate:: Anhydrous sodium perborate monohydrate bleach, empirical formula NaBO₂.H₂O₂
- PB4:: Anhydrous sodium perborate tetrahydrate
- Percarbonate:: Anhydrous sodium percarbonate bleach of empirical formula 2Na₂CO₃.3H₂O₂
- TAED:: Tetra-acetyl ethylene diamine
- CMC:: Sodium carboxymethyl cellulose
- DETPMP:: Diethylene triamine penta (methylene phosphonic acid), marketed by Monsanto under the trade name Dequest 2060
- PVP:: Polyvinylpyrrolidone polymer
- EDDS:: Ethylene diamine-N, N'-disuccinic acid, [S,S] isomer in the form of the sodium salt
- Suds: 25% paraffin wax, melting point 50°C, 17% hydrophobic silica
- Suppressor:: 58% paraffin oil
- Granular Suds: 12% Silicone/silica, 18% stearyl alcohol, 70%
- Suppressor:: starch in granular form
- Sulfate:: Anhydrous sodium sulfate
- HMWPEO:: High molecular weight polyethylene oxide
- TAE 25:: Tallow alcohol ethoxylate (25)

### Detergent Example I

A granular fabric cleaning composition in accordance with the invention may be prepared as follows:

| Component | % |
|---|---|
| Sodium linear C12 alkyl benzene sulfonate | 6.5 |
| Sodium sulphate | 15.0 |
| Zeolite A | 26.0 |
| Sodium nitrilotriacetate | 5.0 |
| Enzyme | 0.1 |
| PVP | 0.5 |
| TAED | 3.0 |
| Boric acid | 4.0 |
| Perborate | 18.0 |
| Phenol sulfonate | 0.1 |
| Minors | up to 100 |

### Detergent Example II

A compact granular fabric cleaning composition (density 800 g/I) in accord with the invention may be prepared as follows:

| Component | % |
|---|---|
| 45AS | 8.0 |
| 25E3S | 2.0 |
| 25E5 | 3.0 |
| 25E3 | 3.0 |
| TFAA | 2.5 |
| Zeolite A | 17.0 |
| NaSKS-6 | 12.0 |
| Citric acid | 3.0 |
| Carbonate | 7.0 |
| MA/AA | 5.0 |
| CMC | 0.4 |
| Enzyme | 0.1 |
| TAED | 6.0 |
| Percarbonate | 22.0 |
| EDDS | 0.3 |
| Granular suds suppressor | 3.5 |
| water/minors | Up to 100 |

### Detergent Example III

Granular fabric cleaning compositions in accordance with the invention that are especially useful in the laundering of coloured fabrics were prepared as follows:

| Component | % | % |
|---|---|---|
| LAS | 10.7 | - |
| TAS | 2.4 | - |
| TFAA | - | 4.0 |
| 45AS | 3.1 | 10.0 |
| 45E7 | 4.0 | - |
| 25E3S | - | 3.0 |
| 68E11 | 1.8 | - |
| 25E5 | - | 8.0 |
| Citrate | 15.0 | 7.0 |
| Carbonate | - | 10.0 |
| Citric acid | 2.5 | 3.0 |
| Zeolite A | 32.1 | 25.0 |
| Na-SKS-6 | - | 9.0 |
| MA/AA | 5.0 | 5.0 |
| DETPMP | 0.2 | 0.8 |
| Enzyme | 0.10 | 0.05 |
| Silicate | 2.5 | - |
| Sulfate | 5.2 | 3.0 |
| PVP | 0.5 | - |
| Poly (4-vinylpyridine)-N-Oxide/ copolymer of vinyl-imidazole and vinyl-pyrrolidone | - | 0.2 |
| Perborate | 1.0 | - |
| Phenol sulfonate | 0.2 | - |
| Water/Minors | Up to 100 | Up to 100 |

### Detergent Example IV

Granular fabric cleaning compositions in accordance with the invention which provide "Softening through the wash" capability may be prepared as follows:

| Component | % | % |
|---|---|---|
| 45AS | - | 10.0 |
| LAS | 7.6 | - |
| 68AS | 1.3 | - |
| 45E7 | 4.0 | - |
| 25E3 | - | 5.0 |
| Coco-alkyl-dimethyl-hydroxyl-ethyl ammonium-chloride | 1.4 | 1.0 |
| Citrate | 5.0 | 3.0 |
| Na-SKS-6 | - | 11.0 |
| Zeolite A | 15.0 | 15.0 |
| MA/AA | 4.0 | 4.0 |
| DETPMP | 0.4 | 0.4 |
| Perborate | 15.0 | - |
| Percarbonate | - | 15.0 |
| TAED | 5.0 | 5.0 |
| Smectite clay | 10.0 | 10.0 |
| HMWPEO | - | 0.1 |
| Enzyme | 0.10 | 0.05 |
| Silicate | 3.0 | 5.0 |
| Carbonate | 10.0 | 10.0 |
| Granular suds suppressor | 1.0 | 4.0 |
| CMC | 0.2 | 0.1 |
| Water/Minors | Up to 100% | Up to 100% |

### Detergent Example V

Heavy duty liquid fabric cleaning compositions in accordance with the invention may be prepared as follows:

| Component | % | % |
|---|---|---|
| LAS acid form | - | 25.0 |
| Citric acid | 5.0 | 2.0 |
| 25AS acid form | 8.0 | - |
| 25AE2S acid form | 3.0 | - |
| 25AE7 | 8.0 | - |
| CFAA | 5.0 | - |
| DETPMP | 1.0 | 1.0 |
| Fatty acid | 8 | - |
| Oleic acid | - | 1.0 |
| Ethanol | 4.0 | 6.0 |
| Propanediol | 2.0 | 6.0 |
| Enzyme | 0.10 | 0.05 |
| Coco-alkyl dimethyl hydroxyl ethyl ammonium chloride | - | 3.0 |
| Smectite clay | - | 5.0 |
| PVP | 2.0 | - |
| Water / Minors | Up to 100 | Up to 100 |

### Powder automatic dish wash composition I

| | |
|---|---|
| Non-ionic surfactant | 0.4 - 2.5% |
| Sodium metasilicate | 0 - 20% |
| Sodium disilicate | 3 - 20% |
| Sodium triphosphate | 20 - 40% |
| Sodium carbonate | 0 - 20% |
| Sodium perborate | 2 - 9% |
| Tetra acetyl ethylene diamine (TAED) | 1 - 4% |
| Sodium sulphate | 5 - 33% |
| Enzymes | 0.0001 - 0.1% |

### Powder automatic dish wash composition II

| | |
|---|---|
| Non-ionic surfactant (e.g. alcohol ethoxylate) | 1 - 2% |
| Sodium disilicate | 2 - 30% |
| Sodium carbonate | 10 - 50% |
| Sodium phosphonate | 0 - 5% |
| Trisodium citrate dehydrate | 9 - 30% |
| Nitrilotrisodium acetate (NTA) | 0 - 20% |
| Sodium perborate monohydrate | 5 - 10% |
| Tetra-acetyl ethylene diamine (TAED) | 1 - 2% |
| Polyacrylate polymer (e.g. maleic acid/acrylic acid copolymer) | 6 - 25% |
| Enzymes | 0.0001 - 0.1% |
| Perfume | 0.1 - 0.5% |
| Water | 5 - 10 |

### Powder automatic dish wash composition III

| | |
|---|---|
| Non-ionic surfactant | 0.5 - 2.0% |
| Sodium disilicate | 25 - 40% |
| Sodium citrate | 30 - 55% |
| Sodium carbonate | 0 - 29% |
| Sodium bicarbonate | 0 - 20% |
| Sodium perborate monohydrate | 0 - 15% |
| Tetra acetyl ethylene diamine (TAED) | 0 - 6% |
| Maleic acid/acrylic acid copolymer | 0 - 5% |
| Clay | 1 - 3% |
| Polyamino acids | 0 - 20% |
| Sodium polyacrylate | 0 - 8% |
| Enzymes | 0.0001 - 0.1% |

### Powder automatic dish wash composition IV

| | |
|---|---|
| Non-ionic surfactant | 1 - 2% |
| Zeolite MAP | 15 - 42% |
| Sodium disilicate | 30 - 34% |
| Sodium citrate | 0 - 12% |
| Sodium carbonate | 0 - 20% |
| Sodium perborate monohydrate | 7 - 15% |
| Tetra acetyl ethylene diamine (TAED) | 0 - 3% |
| Polymer | 0 - 4% |
| Maleic acid/acrylic acid copolymer | 0 - 5% |
| Organic phosphonate | 0 - 4% |
| Clay | 1 - 2% |
| Enzymes | 0.0001 - 0.1% |
| Sodium sulphate | Balance |

### Powder automatic dish wash composition V

| | |
|---|---|
| Non-ionic surfactant | 1 - 7% |
| Sodium disilicate | 18 - 30% |
| Trisodium citrate | 10 - 24% |
| Sodium carbonate | 12 - 20% |
| Monopersulphate (2 KHSO₅.KHSO₄.K₂SO₄) | 15 - 21% |
| Bleach stabilizer | 0.1 - 2% |
| Maleic acid/acrylic acid copolymer | 0 - 6% |
| Diethylene triamine pentaacetate, pentasodium salt | 0 - 2.5% |
| Enzymes | 0.0001 - 0.1% |
| Sodium sulphate, water | Balance |

### Powder and liquid dish wash composition with cleaning surfactant system VI

| Non-ionic surfactant | 0 - 1.5% |
|---|---|
| Octadecyl dimethylamine N-oxide dehydrate | 0 - 5% |
| 80:20 wt.C18/C16 blend of octadecyl dimethylamine N-oxide dihydrate and hexadecyldimethyl amine N-oxide dehydrate | 0 - 4% |
| 70:30 wt. C18/C16 blend of octadecyl bis (hydroxyethyl)amine N-oxide anhydrous and hexadecyl bis (hydroxyethyl)amine N-oxide anhydrous | 0 - 5% |
| C₁₃-C₁₅ alkyl ethoxysulfate with an average degree of ethoxylation of 3 | 0 - 10% |
| C₁₂-C₁₅ alkyl ethoxysulfate with an average degree of ethoxylation of 3 | 0 - 5% |
| C₁₃-C₁₅ ethoxylated alcohol with an average degree of ethoxylation of 12 | 0 - 5% |
| A blend of C₁₂-C₁₅ ethoxylated alcohols with an average degree of ethoxylation of 9 | 0 - 6.5% |
| A blend of C₁₃-C₁₅ ethoxylated alcohols with an average degree of ethoxylation of 30 | 0 - 4% |
| Sodium disilicate | 0 - 33% |
| Sodium tripolyphosphate | 0 - 46% |
| Sodium citrate | 0 - 28% |
| Citric acid | 0 - 29% |
| Sodium carbonate | 0 - 20% |
| Sodium perborate monohydrate | 0 - 11.5% |
| Tetra-acetyl ethylene diamine (TAED) | 0 - 4% |
| Maleic acid/acrylic acid copolymer | 0 - 7.5% |
| Sodium sulphate | 0 - 12.5% |
| Enzymes | 0.0001 - 0.1% |

### Non-aqueous liquid automatic dishwashing composition VII

| | |
|---|---|
| Liquid non-ionic surfactant (e.g. alcohol ethoxylates) | 2.0 - 10.0% |
| Alkali metal silicate | 3.0 - 15.0% |
| Alkali metal phosphate | 20.0 - 40.0% |
| Liquid carrier selected from higher glycols, polyglycols, polyoxides, glycol ethers | 25.0 - 45.0% |
| Stabilizer (e.g. a partial ester of phosphoric acid and a C₁₆-C₁₈ alkanol) | 0.5 - 7.0% |
| Foam suppressor (e.g. silicone) | 0 - 1.5% |
| Enzymes | 0.0001 - 0.1% |

### Non-aqueous liquid dishwashing composition VIII

| | |
|---|---|
| liquid non-ionic surfactant (e.g. alcohol ethoxylates) | 2.0 - 10.0% |
| Sodium silicate | 3.0 - 15.0% |
| Alkali metal carbonate | 7.0 - 20.0% |
| Sodium citrate | 0.0 - 1.5% |
| Stabilizing system (e.g. mixtures of finely divided silicone and low molecular weight dialkyl polyglycol ethers) | 0.5 - 7.0% |
| Low molecule weight polyacrylate polymer | 5.0 - 15.0% |
| Clay gel thickener (e.g. bentonite) | 0.0 - 10.0% |
| Hydroxypropyl cellulose polymer | 0.0 - 0.6% |
| Enzymes | 0.0001 - 0.1% |
| Liquid carrier selected from higher lycols, polyglycols, polyoxides and glycol ethers | Balance |

### Thixotropic liquid automatic dishwashing composition IX

| | |
|---|---|
| C₁₂-C₁₄ fatty acid | 0 - 0.5% |
| Block co-polymer surfactant | 1.5 - 15.0% |
| Sodium citrate | 0 - 12% |
| Sodium tripolyphosphate | 0 - 15% |
| Sodium carbonate | 0 - 8% |
| Aluminium tristearate | 0 - 0.1% |
| Sodium cumene sulfonate | 0 - 1.7% |
| Polyacrylate thickener | 1.32 - 2.5% |
| Sodium polyacrylate | 2.4 - 6.0% |
| Boric acid | 0 - 4.0% |
| Sodium formate | 0 - 0.45% |
| Calcium formate | 0 - 0.2% |
| Sodium n-decydiphenyl oxide disulfonate | 0 - 4.0% |
| Monoethanol amine (MEA) | 0 - 1.86% |
| Sodium hydroxide (50%) | 1.9 - 9.3% |
| 1,2-Propanediol | 0 - 9.4% |
| Enzymes | 0.0001 - 0.1% |
| Suds suppressor, dye, perfumes, water | Balance |

### Liquid automatic dishwashing composition X

| | |
|---|---|
| Alcohol ethoxylate | 0 - 20% |
| Fatty acid ester sulfonate | 0 - 30% |
| Sodium dodecyl sulphate | 0 - 20% |
| Alkyl polyglycoside | 0 - 21% |
| Oleic acid | 0 - 10% |
| Sodium disilicate monohydrate | 18 - 33% |
| Sodium citrate dehydrate | 18 - 33% |
| Sodium stearate | 0 - 2.5% |
| Sodium perborate monohydrate | 0 - 13% |
| Tetra-acetyl ethylene diamine (TAED) | 0 - 8% |
| Maleic acid/acrylic acid copolymer | 4 - 8% |
| Enzymes | 0.0001 - 0.1% |

### Liquid automatic dishwashing composition containing protected bleach particles XI

| | |
|---|---|
| Sodium silicate | 5 - 10% |
| Tetra potassium pyrophosphate | 15 - 25% |
| Sodium triphosphate | 0 - 2% |
| Potassium carbonate | 4 - 8% |
| Protected bleach particles, e.g. chlorine | 5 - 10% |
| Polymeric thickener | 0.7 - 1.5% |
| Potassium hydroxide | 0 - 2% |
| Enzymes | 0.0001 - 0.1% |
| Water | Balance |

XII: Automatic dishwashing compositions as described in I, II, III, IV, VI and X, wherein perborate is replaced by percarbonate.
XIII: Automatic dishwashing compositions as described in I-VI, which additionally contain a manganese catalyst. The manganese catalyst may, e.g., be one of the compounds described in "Efficient manganese catalysts for low-temperature bleaching", Nature, (1994), 369, 637-639.

### MATERIALS AND METHODS

Fungal strain Fusarium Solani
Aspergillus oryzae strain BECh2 (WO 00/39322)
Expression vector pCaHj483 (WO 98/00529)
Aspergillus expression construct pMStr59 (Example VI)

### Protease activity assay.

### AZCL-Casein assay:

Substrate: AZCL-Casein (cross-linked and dyed casein from Megazyme, Cat No: I-AZCAS). Temperature: controlled.

### Assay buffers:

- For pH profile:
   o 100mM succinic acid,
   o 100mM HEPES,
   o 100mM CHES,
   o 100mM CABS, 1mM CaCl2,
   o 150mM KCl,
   o 0.01% Triton X-100
   adjusted with HCl or NaOH to pH-values 3.0; 4.0; 5.0; 6.0; 7.0; 8.0; 9.0; 10.0 and 11.0.
- For others: 0.1 M Borax, pH 9.0.

### Protocol for AZCL-casein assay:

1) 0.02g AZCL-Casein is suspended in 10.0ml assay buffer with gentle stirring.
2) 200 micro L of this suspension is transferred to a tube and placed on ice bath, and then 40 micro L protease sample (diluted in assay buffer) is added to the substrate tube.
3) The assay is initiated by transferring the tube with both substrate and enzyme sample to a thermo mixer, which is pre-warmed for at least 5 minutes under the assay temperature.
4) The tube is incubated for 20 minutes on the thermo mixer at 1200rpm.
5) The incubation is stopped by transferring the tube to an ice bath.
6) Then the tubes are centrifuged in a cold centrifuge for a few minutes.
7) OD595 is measured as a measure for the relative protease activity.

### Protocol for the DMC/TNBS assay:

### Preparation of substrate and reagents:

- DMC substrate solution:
   0.075g DMC (Dimethyl casein, obtainable from Novo Nordisk A/S) is dissolved in 10mL assay buffer (same as AZCL-Casein assay), adjust pH with HCl or NaOH, filtrate with 0.45 micro M membrane and this is used as DMC substrate solution.
- TNBS solution:
   Dilute 17 micro L 1M TNBS (obtainable from Fluka) solution with 5mL 200mM CHES
   (obtainable from Sigma) buffer with pH 9.0, kept on icebath without light exposure.

### Procedure:

1) Transfer 40 micro L DMC substrate solution into a 96 well microtiter plate, after adding 20 micro L protease sample, then mix well and keep on ice bath.
2) The assay is initiated by transferring the microtiter plate to a thermo mixer, which is prewarmed for at least 5 minutes under the assay temperature.
3) The tube is incubated for 20 minutes on the thermo mixer at 600 rpm.
4) The incubation is stopped by transferring the microtiter plate to an ice bath, followed by adding 60 micro L TNBS solution, kept on ice in dark place for 15 minutes.
5) OD405 is read as a measure of relative activity of protease.

### Inhibitors

Streptomyces subtilisin inhibitor (SSI), prepared by Novozymes A/S
Chymotrypsin inhibitor-2 (Cl-2), prepared by Novozymes A/S
Ethylenediaminetetraacetic acid disodium salt (EDTA), from USB life science

### p-Nitroaniline chromogenic substrates

Suc-AAPF-pNA (Sigma S-7388)
Suc -AAPE-pNA (BACHEM L-1710)
Suc -GGF-pNA (Sigma S-1899)
BA-pNA (Benzoyl-DL-Arginine, Sigma B-4875)
Suc -FGL-pNA (Sigma S-6768)

### Example I: Cultivation of fungal strain for enzyme purification and gene cloning.

Fungal strain Fusarium solani containing the gene fragment encoding the protease of the present invention was grown in WB media.
Per 500 ml shake flask:
- 30g wheat bran, and
- 45 mL of the following solution:
   o 0.18g Yeast Extract,
   o 0.045 g KH2PO4,
   o 0.0225g MgSO4.7H2O,
   o 0.675 g glucose, 45 ml tap water,
   autoclaved for 30 minutes at 121 degree) under 25°C for 7 days.

Enzyme extraction was carried out by adding about 150 mL sterilized water and mixing by using a sterilized glass rod, then let it stay overnight at 4°C. Finally supernatant, after filtration and centrifugation, was collected and used as crude protease sample for further purification.

Then mycelium was harvested by directly transferring 10g fermented WB media into a clean plastic bag following by immediately freezing in liquid nitrogen. The frozen mycelium was stored in a minus 80 freezer before use for RNA extraction.

### Example II: Purification of protease from culture broth.

4000mL supernatant of the strain mentioned in Example I was used for purification of protease. The total protein was precipitated with ammonium sulfate (80% saturation).

After centrifugation, the precipitate was re-dissolved in 100 mL of 25 mM phosphate buffer (pH6.0), and then dialyzed with the same buffer.

The dialyzed sample was then filtered through a 0.45 mikrom filter before applying to column for purification. The final volume of the sample was 140 mL.

The filtered sample was applied to a 38 mL SP Sepharose FF column (from Phamacia) equilibrated in 25 mM phosphate buffer, pH6.0, and the proteins was eluted with a linear NaCl gradient (0 -0.3M). Fractions from the column were analyzed for protease activity on AZCL-casein at pH 9.0, with or without SSI pre-inhibition.

Fractions with protease activity not inhibited by SSI were pooled. Then the pooled solution was ultra filtrated with 3k membrane (from Amicon), the concentrated solution was applied to a 180ml Sephacryl 100 column (from Phamacia) equilibrated with 25 mM Tris-HCl, pH7.4.

The proteins were eluted with the same buffer.

After protease activity test (with and without SSI preinhibition), the fractions with protease activity were analyzed by SDS-PAGE and the fractions with purified protease were pooled and used as sample for characterization (see example IV).

### Example III: Gene cloning of the protease.

The gene fragment encoding the protease of the invention was cloned by using RT-PCT technology.

### Extraction of total RNA:

The total RNA was extracted from frozen mycelium by using RNeasy Mini Kit (QIAGEN, Catalogue No. 74904). The total RNA was extracted from 100mg mycelium of the strain described in Example I.

### Specific primer design for cloning:

Specific primer for PCR amplification of trypsin from Fusarium and related fungi was designed based on the conserved region of already known fungal trypsin DNA sequences. The primer sequence (CDS-primer) is shown in SEQ ID NO.:5.

Full length cloning of the protease of the present invention by using 3' RACE Kit: The 3' RACE system (GIBCO, Catalogue No. 18373-019) was used to synthesize cDNA of the protease of the present invention. About 5 micro g total RNA was used as template and Adapter Primer (provided by 3'RACE system) was used to synthesize the first strand of cDNA. Then cDNA of the protease of the present invention was amplified by using specific CDS-primer.

Gel analysis of the PCR product gave a specific band about ∼1kb fragment using CDS-primer and the products were recovered from 1% LMP agarose gel , then purified by incubated in a 70°C followed by using PCR Preps DNA Purification System (Promega, Cat.No.A7170). The purified fragments were ligated to pGEM-T Vector (Promega, Catalogue No. A3600) and then transformed 2-4 micro L ligation products into 50 micro L JM109 high efficiency competent cells by the "heat shock" method.

Transformation culture broth was plated onto the LB plates with ampicillin/IPTG/ X-Gal, and these plates were incubated overnight at 37°C.

Recombinant clones were identified by colour screening on indicator plates and colony PCR screening. The positive clones were inoculated into 3mL LB liquid medium and incubate overnight at 37°C with shaking (∼250 rpm). After precipitating the cells by centrifugation for 5 min at 10,000g, plasmid sample were prepared from the cell precipitate by using Minipreps DNA Purification System (Promega, Catalogue No. A7100). Finally the plasmids were sequenced with BigDye Terminator Cycle Sequencing Ready Reaction Kit (PE) by using AB1377 sequencer and the full length sequence for this gene was successfully obtained.

### Example IV: Characterisation of the protease of the present invention

### Inhibition test:

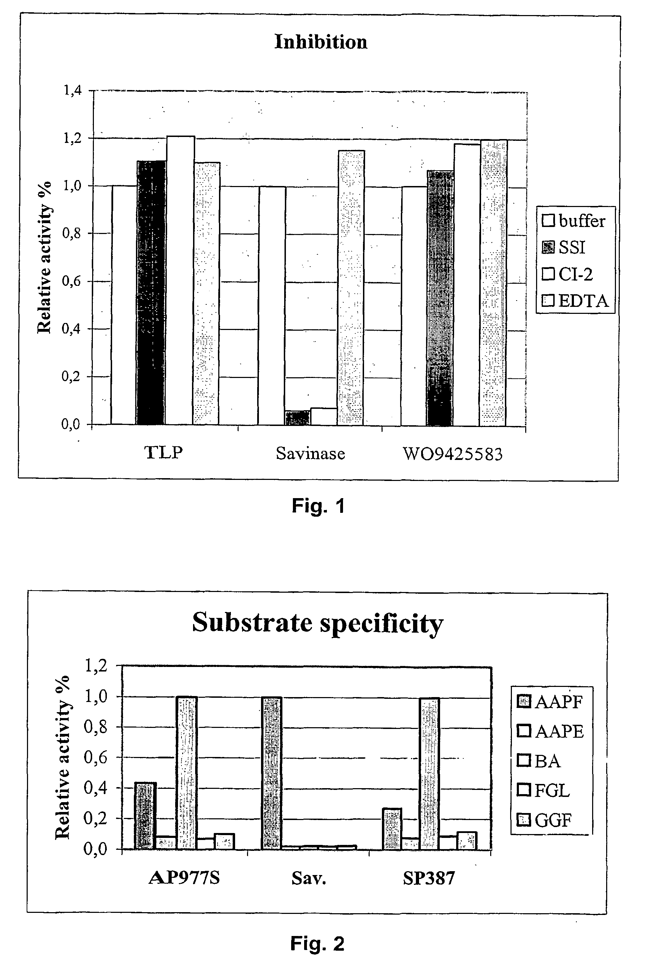

Purified protease of the present invention (see Example II) was tested by using different inhibitors including SSI, C12 and EDTA in AZCL-casein assay, with the trypsin-like protease disclosed in WO 94/25583 and Savinase® as control. The result is illustrated in the figure 1.
Activity of the protease of the present invention is not inhibited by SSI, Cl-2 and EDTA, which means that the protease could be a non subtilisin protease in serine protease family.

### Substrate specificity:

The substrate specificity of the protease of the present invention was tested by using a different pNA substrates including AAPF, AAPE, GGF, BA and FGL, with Savinase® and the protease disclosed in WO9425583 as reference. The result is shown in figure 2.
The protease has the same substrate specificity as trypsin-like protease disclosed in WO9425583 and it is different from the subtilisin protease Savinase®.

### Temperature profile:

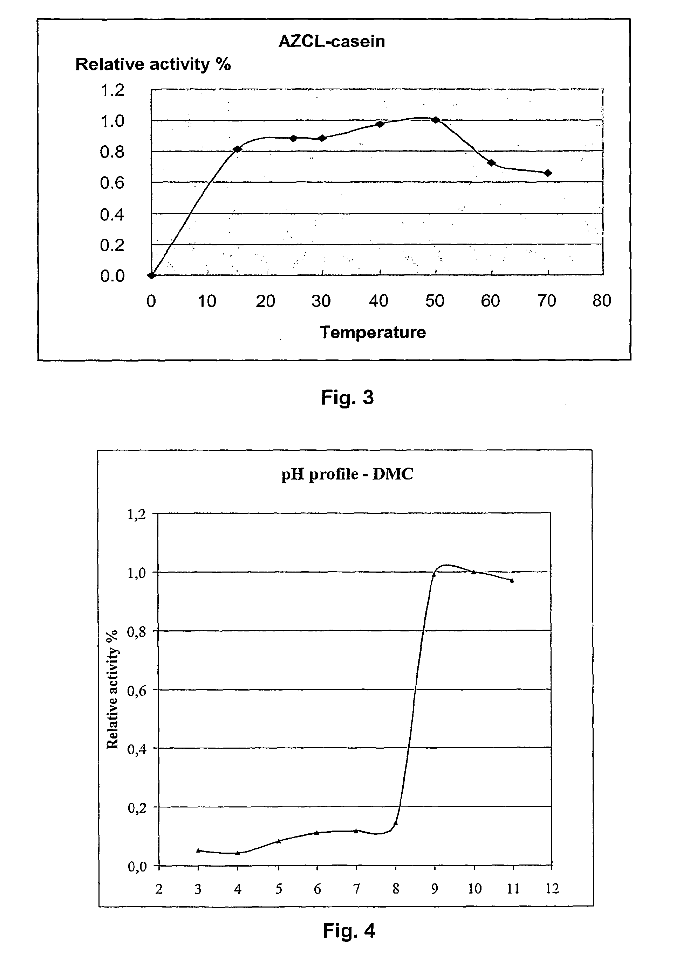

Temperature profile was obtained for the protease of the present invention by using the AZCL-casein assay as described above. The result is shown in figure 3, and it can be seen that it is active from 15°C to 70°C and has an optimal temperature around 40°C to 50°C.

### pH-profile:

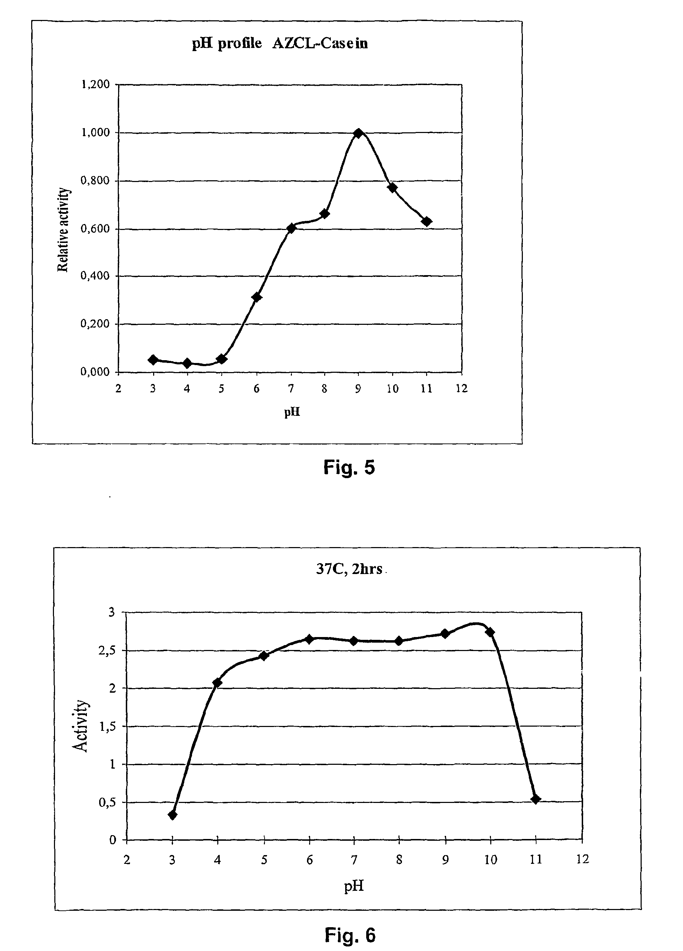

The pH-profile of the protease of the present invention was obtained by using both the AZCL-casein assay and the DMC-assay as described in the section "Protease activity assay".
The profile is shown in figure 5 (AZCL casein assay) and figure 4 (DMC assay).
The protease exhibits activity from pH 3 to pH 11 with optimal activity around pH 9. In both assays the protease showed high activity up to pH 11.

### pH-stability:

For pH stability test, 15 micro L enzyme sample was mixed with 200 micro L buffer pH 3,4,5,6,7,8,9,10,11 and was incubated at 37°C for 2 hours. Subsequently the enzyme activity was assayed by using AZCL-casein assay as described above in the section "Protease activity assay". The result is shown in figure 6. It can be seen that the protease of the present invention is stable in a wide pH-range from pH 4 to pH 10.

### Example V: Stability in detergent.

Protease is diluted in de-ionised water and mixed with a solution of Attack detergent from KAO (1.4 g/l, 6°dH (CaCl2:MgCl2 2:1) in de-ionised water, no heat inactivation) giving a final detergent concentration of 0.7 g/l (3°dH) and a relevant protease content. A protease dosage corresponding to 25-50 mA at OD280/ml in the mixture is normally. Triple replicates are used.

The mixture is either incubated for 30 min at 20°C, 25°C, 30°C, and 35°C before determination of activity in DMC/TNBS assay or activity is measured immediately as a reference.

The residual activity as shown in table below is calculated as activity of stored sample relative to activity of reference sample.

| Residual activity (%) as a function of temperature | | | | |
|---|---|---|---|---|
| Temperature (°C) | 20 | 25 | 30 | 35 |
| WO 9425583 | 82 | 60 | 8 | 4 |
| Enzyme of the present invention. | 69 | 72 | 72 | 71 |

As can be seen from the data the protease of the present invention exhibits pronounced improvement in stability compared to the protease disclosed in WO 9425583.

### Example VI Expression of a trypsin from Fusarium solani in Aspergillus oryzae

The nucleotide sequence encoding the Fusarium solani trypsin, Sequence ID No: 1, was used to design primers for PCR amplification of the gene with appropriate restriction sites added to the primer ends to facilitate cloning into an expression vector (see SEQ ID NO: 3 and SEQ ID NO: 4; primer sequences).

PCR amplification was performed using Pfu Turbo DNA polymerase (Stratagene, La Jolla, California, USA) following the manufacturers instructions and using the cDNA clone described in example III as a template and an annealing temperature of 58°C and an extension time of 1 minute for 30 cycles. A single PCR product was obtained, restricted with Xhol and cloned into the Aspergillus expression vector pCaHj483 (WO 98/00529) restricted with Nrul and Xhol using standard techniques. The expression vector pCaHj483 has sequences for selection and propogation in E. coli, and selection and expression in Aspergillus.

Specifically, selection in Aspergillus is facilitated by the amdS gene of Aspergillus nidulans, which allows the use of acetamide as a sole nitrogen source. Expression in Aspergillus is mediated by the neutral amylase II (NA2) promoter from Aspergillus niger which is fused to the 5' leader sequence of the triose phosphate isomerase (tpi) encoding-gene from Aspergillus nidulans, and the terminator from the amyloglucosidase-encoding gene from Aspergillus niger. The trypsin-encoding gene of the resulting Aspergillus expression construct, pMStr59, was sequenced and the sequence was compared to that determined previously to confirm that no errors were introduced during PCR amplification.

Aspergillus oryzae strain BECh2 (WO 00/39322) was transformed with pMStr59 using standard techniques (Christensen, T. et al., (1988), Biotechnology 6, 1419-1422). Transformants were cultured by the method described in WO 94/26925, using 100ml of FG4P medium amended with 5.0AU/l of Neutrase 1.5 MG (Novozymes A/S, Bagsvaerd, Denmark) in 250 ml flasks. Neutrase was dissolved in universal buffer at pH 6.5 and filter sterilized before addition. Cultures were shaken at 270 RPM at 26°C for 4 days.

Expression of the Fusarium trypsin was assayed as described in WO 94/26925, using N-Benzoyl-L-arginine-p-nitroanilide hydrochloride (L-BA-pNA) as a substrate and a tris buffer, pH 8.0, of the following composition: 25mM tris, 1 mM CaCl₂ and 150mM KCI.

### Universal buffer:

100mM succinic acid
100mM HEPES
100mM CHES
100mM CABS
1mM CaCl₂
150mM KCI
0.01% Triton X-100

### EXAMPLE VII - "Model Detergent Wash Performance Test"

In order to assess the wash performance of subtilases in a standard detergent composition, standard washing experiments may be performed using the below experimental conditions:

| Detergent: | Model detergent |
|---|---|
| Detergent dosage | 4.0 g/l |
| pH | 10.1 |
| Wash time | 20 min |
| Temperature: | 30°C |
| Water hardness: | 15°dH |
| Enzyme concentration: | 10 nm (in the detergent solution) |
| Test system: | 10 ml beakers with a stirring rod |
| Textile/volume: | 5 textile pieces (Ø 2.5 cm)/50 ml detergent solution |
| Test material: | EMPA 117 |

The composition of the model detergent is as follows:

| | |
|---|---|
| 6.2% | LAS (Nansa 80S) |
| 2% | Sodium salt of C₁₆-C₁₈ fatty acid |
| 4% | Non-ionic surfactant (Plurafax LF404) |
| 22% | Zeolite P |
| 10.5% | Na₂CO₃ |
| 4% | Na₂Si₂O₅ |
| 2% | Carboxymethylcellulose (CMC) |
| 6.8% | Acrylate liquid CP5 40% |
| 20% | Sodium perborate (empirical formula NaBO₂.H₂O₂) |
| 0.2% | EDTA |
| 21% | Na₂SO₄ Water (balance) |

pH of the detergent solution is adjusted to 10.1 by addition of HCl or NaOH. Water hardness is adjusted to 15°dH by addition of CaCl₂ and MgCl₂ (Ca²⁺:Mg²⁺ = 4:1) to the test system. After washing the textile pieces are flushed in tap water and air-dried.

Measurement of the reflectance (R_{subtilase}) on the test material is performed at 460 nm using a Macbeth ColorEye 7000 photometer (Macbeth, Division of Kollmorgen Instruments Corporation, Germany). The measurements are performed in accordance with the manufacturer's protocol.

In order to determine a blank value, a similar wash experiment is performed without addition of enzyme. The subsequent measurement of the reflectance (R_{blank}) is performed as described right above.

A reference experiment is then performed as described above, wherein the wash performance of a relevant protease is tested. The subsequent measurement of the reflectance (Rₛₐᵥᵢₙₐₛₑ) is performed as described right above.

### DEPOSIT OF BIOLOGICAL MATERIAL

The following biological material has been deposited under the terms of the Budapest Treaty with the Deutsche Sammlung von Mikroorganismen und Zellkulturen GmbH, Mascheroder Weg 1 B, D-38124 Braunschweig, Germany, and given the following accession numbers:

| Deposit | Accession Number | Date of deposit |
|---|---|---|
| Escherichia coli NN049696 | DSM 15940 | 2003-09-22 |

## Claims

1. A protease selected from the group consisting of
a. a protease comprising an amino acid sequence which has at least 80% identity with the amino acid sequence shown as amino acids 1 to 226 of SEQ ID NO: 2; and
b. a protease which is encoded by a nucleic acid sequence which has at least 80% identity with the nucleic acid sequence shown as nucleic acid 52-804 of SEQ ID NO: 1,
where the protease - when tested as in "Example V Stability in detergent" - has a residual activity of at least 50% after storage at 35°C.

2. A protease according to claim 1 having an amino acid sequence which has more than 85.0%, or more than 90.0%, or more than 92.0%, or more than 94.0%, or more than 96.0%, or more than 97.0%, or more than 98.0%, or more than 99.0% identity with the amino acid sequence shown as amino acids 1 to 226 of SEQ ID NO: 2.

3. A protease according to any of the preceding claims, wherein the protease is a variant of a protease having the amino acid sequence shown as amino acids -25 to 226 of SEQ ID NO: 2 comprising a substitution, deletion, and/or insertion of one or more amino acid residues.

4. A protease according to claim 1, which is encoded by the gene encoding the subtilase having the amino acid sequence shown in SEQ ID NO: 2 and inserted into *Escherichia coli* DSM 15940, or a variant thereof having more than 80% identity to amino acids 1 to 226 of SEQ ID NO: 2.

5. A protease according to claim 4 having more than 85.0%, or more than 90.0%, or more than 92.0%, or more than 94.0%, or more than 96.0%, or more than 97.0%, or more than 98.0%, or more than 99.0% identity with amino acids 1 to 226 of SEQ ID NO: 2.

6. An isolated nucleic acid sequence comprising a nucleic acid sequence which encodes for the protease defined in any of the preceding claims.

7. A nucleic acid sequence according to claim 6, having a nucleic acid sequence which has at least 86%, such as at least 87%, e.g. at least 88%, preferably at least 89%, such as ate least 90%, e.g. at least 91%, more preferably at least 92%, such as at least 93%, e.g. at least 94%, most preferably at least 95%, such as at least 96%, e.g. at least 97%, in particular at least 98%, preferably at least 99% identity with the nucleic acid sequence shown as nucleotides 52 to 804 of SEQ ID NO: 1.

8. A nucleic acid construct comprising the nucleic acid sequence of any of claims 6-7 operably linked to one or more control sequences capable of directing the expression of the protease in a suitable host.

9. A recombinant expression vector comprising the nucleic acid construct of claim 8, a promoter, and transcriptional and translational stop signals.

10. A recombinant host cell comprising the nucleic acid construct of claim 8.

11. A host cell according to claim 10, which is a fungus or yeast, preferably a filamentous fungus, especially an Aspergillus.

12. A host cell according to claim 11 which is an *Aspergillus oryzae.*

13. A host cell according to claim 10, which is a bacterium, preferably a Bacillus, especially a *Bacillus lentus.*

14. A method for producing the protease according to any of claims 1-5, the method comprising:
a. cultivating a recombinant host cell as defined in any of claims 11-13 under conditions conducive to the production of the protease; and
b. recovering the protease.

15. A cleaning or detergent composition, preferably a laundry or dishwash composition, comprising the protease according to any of claims 1-5.

16. A composition according to claim 15, which additionally comprises a cellulase, lipase, cutinase, oxidoreductase, another protease, an amylase or a mixture thereof.

17. Use of a protease as defined in any of claims 1-5 in a cleaning or detergent composition.

18. A method for cleaning or washing a hard surface or laundry, the method comprising contacting the hard surface or the laundry with the composition defined in claim 16.

## Patentansprüche

1. Protease ausgewählt aus der Gruppe bestehend aus:
a. einer Protease umfassend eine Aminosäuresequenz, die mindestens 80% Identität mit der als Aminosäuren 1 bis 226 von SEQ ID NO: 2 gezeigten Aminosäuresequenz aufweist; und
b. einer Protease, die durch eine Nukleinsäuresequenz kodiert wird, die mindestens 80% Identität mit der als Nukleinsäure 52-804 von SEQ ID NO: 1 gezeigten Nukleinsäuresequenz aufweist,
wobei die Protease - wenn wie in "Beispiel V: Stabilität in Detergens" getestet- eine Restaktivität von mindestens 50% nach Lagerung bei 35°C aufweist.

2. Protease nach Anspruch 1 mit einer Aminosäuresequenz, die mehr als 85,0%, oder mehr als 90,0%, oder mehr als 92,0%, oder mehr als 94,0%, oder mehr als 96,0%, oder mehr als 97,0%, oder mehr als 98,0%, oder mehr als 99,0% Identität mit der als Aminosäuren 1 bis 226 von SEQ ID NO: 2 gezeigten Aminosäuresequenz aufweist.

3. Protease nach einem beliebigen der vorangehenden Ansprüche, wobei die Protease eine Variante einer Protease mit der als Aminosäuren -25 bis 226 von SEQ ID NO: 2 gezeigten Aminosäuresequenz ist, umfassend eine Substitution, Deletion, und/oder Insertion von einem oder mehreren Aminosäureresten.

4. Protease nach Anspruch 1, die durch das Gen kodiert ist, das die Subtilase mit der in SEQ ID NO: 2 gezeigten Aminosäuresequenz kodiert und in *Escherichia coli* DSM 15940 insertiert ist, oder eine Variante davon mit mehr als 80% Identität zu Aminosäuren 1 bis 226 von SEQ ID NO: 2.

5. Protease nach Anspruch 4 mit mehr als 85,0% oder mehr als 90,0% oder mehr als 92,0%, oder mehr als 94,0% oder mehr als 96,0% oder mehr als 97,0% oder mehr als 98,0% oder mehr als 99,0% Identität mit Aminosäuren 1 bis 226 von SEQ ID NO: 2.

6. Isolierte Nukleinsäuresequenz umfassend eine Nukleinsäuresequenz, die die in einem beliebigen der vorangehenden Ansprüche definierte Protease kodiert.

7. Nukleinsäuresequenz nach Anspruch 6 mit einer Nukleinsäuresequenz, die mindestens 86%, wie mindestens 87%, z. B. mindestens 88%, bevorzugt mindestens 89%, wie mindestens 90%, z. B. mindestens 91%, weiter bevorzugt mindestens 92%, wie mindestens 93%, z. B. mindestens 94%, am meisten bevorzugt mindestens 95%, wie mindestens 96%, z. B. mindestens 97%, insbesondere mindestens 98%, bevorzugt mindestens 99% Identität mit der als Nukleotide 52 bis 804 von SEQ ID NO: 1 gezeigten Nukleinsäuresequenz aufweist.

8. Nukleinsäurekonstrukt umfassend die Nukleinsäuresequenz gemäß einem beliebigen der Ansprüche 6-7, funktionsfähig verknüpft mit einer oder mehreren Kontrollsequenzen, die fähig ist/sind, die Expression der Protease in einem geeigneten Wirt zu steuern.

9. Rekombinanter Expressionsvektor umfassend das Nukleinsäurekonstrukt gemäß Anspruch 8, einen Promotor und transkriptionale und translationale Stoppsignale.

10. Rekombinante Wirtszelle umfassend das Nukleinsäurekonstrukt gemäß Anspruch 8.

11. Wirtszelle gemäß Anspruch 10, die ein Pilz oder eine Hefe ist, bevorzugt ein filamentöser Pilz, insbesondere ein Aspergillus.

12. Wirtszelle nach Anspruch 11, die ein *Aspergillus oryzae* ist.

13. Wirtszelle nach Anspruch 10, die ein Bakterium ist, bevorzugt ein Bacillus, insbesondere ein *Bacillus lentus.*

14. Verfahren zum Herstellen der Protease gemäß einem beliebigen der Ansprüche 1-5, wobei das Verfahren umfasst:
a. Kultivieren einer rekombinanten Wirtszelle wie in einem beliebigen der Ansprüche 11-13 definiert unter Bedingungen, die zur Herstellung der Protease förderlich sind; und
b. Gewinnen der Protease.

15. Eine Reinigungs- oder Detergenszusammensetzung, bevorzugt eine Waschmittel- oder Geschirrspülzusammensetzung, umfassend die Protease gemäß einem beliebigen der Ansprüche 1-5.

16. Zusammensetzung nach Anspruch 15, die zusätzlich eine Cellulase, Lipase, Cutinase, Oxidoreduktase, eine andere Protease, eine Amylase oder eine Mischung davon umfasst.

17. Verwendung einer Protease wie in einem beliebigen der Ansprüche 1-5 definiert in einer Reinigungs- oder Detergenszusammensetzung.

18. Verfahren zum Reinigen oder Waschen einer harten Oberfläche oder von Wäsche, wobei das Verfahren Inkontaktbringen der harten Oberfläche oder der Wäsche mit der in Anspruch 16 definierten Zusammensetzung umfasst.

## Revendications

1. Protéase choisie dans le groupe consistant en
a. une protéase comprenant une séquence d'acides aminés qui présente une identité d'au moins 80% avec la séquence d'acides aminés représentée par les acides aminés 1 à 226 de SEQ ID NO: 2; et
b. une protéase codée par une séquence d'acide nucléique qui présente une identité d'au moins 80% avec la séquence d'acide nucléique représentée par les nucléotides 52-804 de SEQ ID NO: 1,
où la protéase - quand elle est testée comme dans «Exemple V Stabilité dans un détergent» - a une activité résiduaire d'au moins 50% après un stockage à 35°C.

2. Protéase selon la revendication 1 ayant une séquence d'acides aminés qui présente une identité supérieure à 85,0%, ou supérieure à 90,0%, ou supérieure à 92,0%, ou supérieure à 94,0%, ou supérieure à 96,0%, ou supérieure à 97,0%, ou supérieure à 98,0%, ou supérieure à 99,0% avec la séquence d'acides aminés représentée par les acides aminés 1 à 226 de SEQ ID NO: 2.

3. Protéase selon l'une quelconque des revendications précédentes, dans laquelle la protéase est un variant d'une protéase ayant la séquence d'acides aminés représentée par les acides aminés 25 à 226 de SEQ ID NO: 2 comprenant une substitution, une délétion, et/ou une insertion d'un ou de plusieurs résidus d'acides aminés.

4. Protéase selon la revendication 1, codée par le gène codant pour la subtilase ayant la séquence d'acides aminés représentée par SEQ ID NO: 2 et insérée dans *Escherichia coli* DSM 15940, ou un variant de celle-ci présentant une identité supérieure à 80% avec les acides aminés 1 à 226 de SEQ ID NO: 2.

5. Protéase selon la revendication 4 présentant une identité supérieure à 85,0%, ou supérieure à 90,0%, ou supérieure à 92,0%, ou supérieure à 94,0%, ou supérieure à 96,0%, ou supérieure à 97,0%, ou supérieure à 98,0%, ou supérieure à 99,0% avec les acides aminés 1 à 226 de SEQ ID NO: 2.

6. Séquence d'acide nucléique isolée comprenant une séquence d'acide nucléique qui code pour la protéase définie dans l'une quelconque des revendications précédentes.

7. Séquence d'acide nucléique selon la revendication 6, ayant une séquence d'acide nucléique qui présente une identité d'au moins 86%, telle que d'au moins 87%, par exemple, d'au moins 88%, de préférence, d'au moins 89%, telle que d'au moins 90%, par exemple, d'au moins 91 %, plus préférablement d'au moins 92%, telle que d'au moins 93%, par exemple, d'au moins 94%, le plus préférablement, d'au moins 95%, telle que d'au moins 96%, par exemple, d'au moins 97%, en particulier, d'au moins 98%, de préférence, d'au moins 99% avec la séquence d'acide nucléique représentée par les nucléotides 52 à 804 de SEQ ID NO: 1.

8. Construction d'acide nucléique comprenant la séquence d'acide nucléique selon l'une quelconque des revendications 6-7 liée de manière opérationnelle à une ou plusieurs séquences contrôle capables de diriger l'expression de la protéase chez un hôte convenable.

9. Vecteur d'expression recombinant comprenant la construction d'acide nucléique selon la revendication 8, un promoteur, et des signaux de terminaison de la transcription et de la traduction.

10. Cellule hôte recombinante comprenant la construction d'acide nucléique selon la revendication 8.

11. Cellule hôte selon la revendication 10, laquelle est un champignon ou une levure, de préférence, un champignon filamenteux, notamment un Aspergillus.

12. Cellule hôte selon la revendication 11 laquelle est un *Aspergillus oryzae.*

13. Cellule hôte selon la revendication 10, laquelle est une bactérie, de préférence, un Bacillus, et notamment, un *Bacillus lentus.*

14. Procédé de production de la protéase selon l'une quelconque des revendications 1-5, le procédé comprenant:
a. la culture d'une cellule hôte recombinante comme définie dans l'une quelconque des revendications 11-13 dans des conditions aptes à induire la production de la protéase; et
b. la récupération de la protéase.

15. Composition nettoyante ou détergente, de préférence, composition pour laver le linge ou la vaisselle, comprenant la protéase selon l'une quelconque des revendications 1-5.

16. Composition selon la revendication 15, qui comprend en plus une cellulase, une lipase, une cutinase, une oxydoréductase, une autre protéase, une amylase ou un mélange de celles-ci.

17. Utilisation d'une protéase comme définie dans l'une quelconque des revendications 1-5 dans une composition nettoyante ou détergente.

18. Procédé de nettoyage ou de lavage d'une surface dure ou du linge, le procédé comprenant la mise en contact de la surface dure ou du linge avec la composition définie dans la revendication 16.
